(19) Europäisches Patentamt
European Patent Office
Office européen des brevets

(11) EP 1 565 148 B1

(12) FASCICULE DE BREVET EUROPEEN

(45) Date de publication et mention de la délivrance du brevet:
**30.03.2011 Bulletin 2011/13**

(21) Numéro de dépôt: **03798228.7**

(22) Date de dépôt: **26.09.2003**

(51) Int Cl.:
***A61K 8/90*** (2006.01) ***A61K 8/81*** (2006.01)
***A61K 8/26*** (2006.01) ***C08F 265/06*** (2006.01)
***A61Q 3/02*** (2006.01) ***A61Q 1/06*** (2006.01)
***C08L 51/00*** (2006.01) ***C08L 53/00*** (2006.01)

(86) Numéro de dépôt international:
**PCT/FR2003/002842**

(87) Numéro de publication internationale:
**WO 2004/028488 (08.04.2004 Gazette 2004/15)**

# (54) COMPOSITION LIQUIDE BRILLANTE COMPRENANT UN POLYMÈRE SÉQUENCÉ

BLOCKPOLYMER ENTHALTENDE FLÜSSIGE GLÄNZENDE ZUSAMMENSETZUNG

GLOSSY LIQUID COMPOSITION COMPRISING A SEQUENCED POLYMER

(84) Etats contractants désignés:
**AT BE BG CH CY CZ DE DK EE ES FI FR GB GR HU IE IT LI LU MC NL PT RO SE SI SK TR**

(30) Priorité: **26.09.2002 FR 0211949**
**20.12.2002 FR 0216437**
**21.05.2003 FR 0306121**

(43) Date de publication de la demande:
**24.08.2005 Bulletin 2005/34**

(73) Titulaire: **L'Oréal**
**75008 Paris (FR)**

(72) Inventeurs:
- **BLIN, Xavier**
**75015 Paris (FR)**
- **FERRARI, Véronique**
**F-94700 Maisons-Alfort (FR)**

(74) Mandataire: **Kromer, Christophe**
**L'Oréal**
**D.I.P.I.**
**25-29 Quai Aulagnier**
**92600 Asnières (FR)**

(56) Documents cités:
**EP-A- 1 082 953**
**EP-A- 1 192 930**
**WO-A-02/05765**
**DE-A- 10 029 697**
**FR-A- 2 296 402**
**FR-A- 2 803 743**
**FR-A- 2 809 306**
**FR-A- 2 832 719**
**US-A- 5 110 582**
**US-A- 6 106 820**
**US-A- 6 153 206**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se rapporte à une composition cosmétique liquide de maquillage ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable contenant un polymère séquencé particulier.

Il existe de nombreuses compositions cosmétiques pour lesquelles les propriétés de brillance du film déposé, après application sur les matières kératiniques (peau, lèvres, phanères) sont très importantes. On peut citer par exemple les rouges à lèvres, les vernis à ongles ou encore certains produits capillaires et certains mascaras.

Il est connu des formulateurs de produits cosmétiques que cette propriété de brillance est favorisé lorsque l'on utilise des huiles caractérisées par une viscosité et un indice de réfraction élevés et qui possèdent, en outre, de bonnes propriétés de dispersion des pigments ou des charges lorsque ces derniers sont présents dans la composition.

Dans cette optique, le formulateur dispose de plusieurs types de matières premières, comme des polymères huileux tels que les polybutènes, vendus notamment sous la référence Indopol. H100, H300 et H1500 par la Société Amoco, qui ont une viscosité très élevée mais qui présentent cependant l'inconvénient d'être à la fois très collants et de posséder des propriétés de dispersion des pigments relativement faibles, ce qui limite leur utilisation.

La présente invention a pour but de proposer une nouvelle voie de formulation d'un produit cosmétique, en particulier un produit de maquillage, qui permette de bonnes propriétés de brillance.

En particulier, le produit de l'invention permet l'obtention de dépôts continus sur la peau ou les lèvres, de bonne couvrance, ayant un aspect très brillant, adapté au désir de la consommatrice, ne migrant pas, ne transférant pas et de bonne tenue, non huileux, ne desséchant pas la peau, les cheveux ou les lèvres sur lesquelles il est appliqué, aussi bien lors de l'application qu'au cours du temps. Il présente, en outre de bonnes propriétés de stabilité et permet ainsi une application homogène et esthétique.

L'invention a pour objet une composition liquide brillante de soin ou de maquillage de la peau et/ou des lèvres et/ou des phanères contenant un polymère séquence qui permet de remédier aux inconvénients des compositions brillantes connues. De façon surprenante, les inventeurs ont trouvé que l'utilisation d'une quantité suffisante d'un polymère séquence particulier permet d'obtenir une composition brillante et qui, en outre, est de bonne tenue.

La composition de l'invention peut en particulier constituer un produit capillaire ou un produit de maquillage du corps, des lèvres ou des phanères d'êtres humains ayant des propriétés de soin et/ou de traitement. Elle constitue notamment un rouge à lèvres ou un brillant à lèvres, un fard à paupières, un produit pour tatouage, un mascara, un eye-liner, un vernis à ongles, un produit de bronzage artificiel de la peau, une crème de soin ou de protection éventuellement teintée, un produit de coloration ou de soin des cheveux.

De façon plus précise, l'invention a pour objet une composition cosmétique liquide contenant un milieu liquide organique cosmétiquement acceptable, et un polymère séquence éthylénique linéaire filmogène non élastomère,
ledit polymère séquence contenant des première et deuxième séquences reliées entre elles par un segment intermédiaire qui est un polymère statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence étant choisie dans une catégorie a), b) ou c) différente de la première séquence ;
et ledit polymère séquencé ayant un indice de polydispersité 1 supérieur à 2,8,
ledit polymère étant tel, que lorsqu'il est en quantité suffisante dans la composition, la brillance moyenne mesurée à 20° d'un dépôt de ladite composition, une fois étalée sur un support, est de préférence supérieure ou égale à 30 sur 100.

L'invention a également pour objet une composition cosmétique liquide contenant un milieu liquide organique cosmétiquement acceptable, et un polymère séquencé éthylénique linéaire filmogène exempt de motif styrène,
ledit polymère séquencé contenant des première et deuxième séquences reliées entre elles par un segment intermédiaire qui est un polymère statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence étant choisie dans une catégorie a), b) ou c) différente de la première séquence ;
et ledit polymère séquencé ayant un indice de polydispersité 1 supérieur à 2,8,
ledit polymère étant tel, que lorsqu'il est en quantité suffisante dans la composition, la brillance moyenne mesurée à 20° d'un dépôt de ladite composition, une fois étalée sur un support, est de préférence supérieure ou égale à 30 sur 100.

Le brevet US 6153206 décrit une composition cosmétique comprenant un polymère contenant des unités répétitives ayant une Tg allant de -10 à 75 °C et des unités répétitives ayant une Tg allant de 76 à 120 °C. Il décrit également des polymères blocs comprenant des blocs de poly méthyl méthacrylate et des blocs de polyisobutyl méthacrylate, et également des polymères triblocs comprenant des blocs de poly méthyl méthacrylate, des blocs de polyisobutyl méthacrylate et des blocs de d'éthyl methacrylate. Les documents FR-A-2809306, WO 03/046032 décrivent des compositions cosmétiques comprenant un polymère éthylénique séquencé. Dans les exemples, le seul polymère exemplifié à un indice de polydispersité respectivement égal à 2,2 et 1,16.

Par "composition liquide", on entend une composition qui, à 25°C et pression atmosphérique, prend la forme du récipient dans laquelle elle est versée.

De préférence, la composition selon l'invention est une composition non rincée.

## Brillance moyenne de la composition

Par « brillance moyenne », on désigne la brillance telle qu'elle peut être mesurée à l'aide d'un brillancemètre, de manière conventionnelle par la méthode suivante.

Sur une carte de contraste de marque LENETA et de référence FORM 1A PENOPAC, on étale une couche comprise entre 50 µm et 150 µm d'épaisseur de la composition à l'aide d'un étaleur automatique. La couche recouvre au moins le fond blanc de la carte. On laisse sécher le dépôt 24 heures à une température de 30°C, puis on procède à la mesure de la brillance à 20° sur le fond blanc à l'aide d'un brillancemètre de marque BYK GARDNER et de référence microTRI-GLOSS.

Cette mesure (comprise entre 0 et 100) est répétée au moins trois fois, et la brillance moyenne est la moyenne des au moins trois mesures effectuées.

La brillance moyenne de la composition mesurée à 20° est avantageusement supérieure ou égale à 30, mieux supérieure ou égale à 35, mieux encore supérieure ou égale à 40, mieux encore supérieure ou égale à 45, mieux encore supérieure ou égale à 50 sur 100, mieux encore, supérieure ou égale à 55, mieux encore, supérieure ou égale à 60, mieux encore, supérieure ou égale à 65, mieux encore, supérieure ou égale à 70 ou mieux encore, supérieure ou égale à 75 sur 100. Pour certaines compositions selon l'invention, telles que des vernis à ongles, la brillance mesurée à 20° peut être supérieure ou égale à 70, voire 80 sur 100.

De préférence, la brillance moyenne de la composition, une fois étalée sur un support, mesurée à 60° est supérieure ou égale à 50, mieux encore, supérieure ou égale à 60, mieux encore, supérieure ou égale à 65, mieux encore, supérieure ou égale à 70, mieux encore, supérieure ou égale à 75, mieux encore, supérieure ou égale à 80, mieux encore, supérieure ou égale à 85 ou mieux encore, supérieure ou égale à 90 sur 100.

On procède à la mesure de la brillance moyenne à 60° comme suit. La brillance peut être mesurée à l'aide d'un brillancemètre, de manière conventionnelle par la méthode suivante.

Sur une carte de contraste de marque LENETA et de référence FORM 1A PENOPAC, on étale une couche comprise entre 50 µm et 150 µm d'épaisseur de la composition à l'aide d'un étaleur automatique. La couche recouvre au moins le fond blanc de la carte. On On laisse sécher le dépôt 24 heures à une température de 30°C, puis on procède à la mesure de la brillance à 60° sur le fond blanc à l'aide d'un brillancemètre de marque BYK GARDNER et de référence microTRI-GLOSS.

Cette mesure (comprise entre 0 et 100) est répétée au moins trois fois, et la brillance moyenne est la moyenne des au moins trois mesures effectuées.

Selon un mode de mise en oeuvre, la brillance moyenne de la composition mesurée à 20° est de préférence supérieure ou égale à 35, de préférence 40, 45 ou 50 sur 100, et/ou la brillance moyenne de la composition mesurée à 60° est de préférence supérieure ou égale à 65, 70 ou 75 sur 100. Dans ce mode de mise en oeuvre, la composition constitue avantageusement un rouge à lèvres liquide.

Selon un mode de mise en oeuvre, la brillance moyenne de la composition mesurée à 20° est de préférence supérieure ou égale à 60, de préférence 65, 70 ou 75 sur 100, et/ou la brillance moyenne de la composition mesurée à 60° est de préférence supérieure ou égale à 80, 85 ou 90 sur 100. Dans ce mode de mise en oeuvre, la composition constitue avantageusement un vernis à ongles.

L'invention se rapporte également à une composition cosmétique comprenant un milieu liquide organique et au moins un polymère séquencé tel que décrit ci-après.

L'invention se rapporte aussi à un procédé de maquillage de la peau et/ou des lèvres et/ou des phanères consistant à appliquer sur la peau et/ou les lèvres et/ou les phanères la composition tel que définie précédemment.

La composition selon l'invention peut être appliquée sur la peau aussi bien du visage que du cuir chevelu et

du corps, des muqueuses comme les lèvres, de l'intérieur des paupières inférieures, et des phanères comme les ongles, les cils, les cheveux, les sourcils, voire les poils.

L'invention se rapporte également à l'utilisation d'un polymère séquencé en une quantité suffisante dans une composition cosmétique pour conférer de la brillance et/ou de la tenue à un dépôt de ladite composition.

L'invention se rapporte également à l'utilisation cosmétique de la composition définie ci-dessus pour améliorer la brillance du maquillage sur la peau et/ou les lèvres et/ou les phanères.

La composition contient avantageusement une proportion moindre d'huiles traditionnellement utilisées pour conférer de la brillance, lesquelles huiles sont généralement collantes.

La composition selon l'invention contient avantageusement moins de 30 %, de préférence moins de 25%, moins de 20%, et mieux moins de 15% d'au moins une huile brillante.

Par « huile », on entend un composé non miscible à l'eau en toute proportion, liquide à température ambiante (25°C) et pression atmosphérique (760 mm de Hg).

Par "huile brillante" on entend une huile dont la masse molaire va, de préférence, de 650 à 10000 g/mol, et de préférence de 750 à 7500 g/mol.

L'huile de masse molaire allant de 650 à 10000 g/mol peut être choisie parmi :

- les polymères lipophiles tels que :
  - les polybutylènes tels que L'INDOPOL H-100 (de masse molaire ou MM=965 g/mol), L'INDOPOL H-300 (MM=1340 g/mol), L'INDOPOL H-1500 (MM=2160g1mol) commercialisés ou fabriqués par la société AMOCO,
  - les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
  - les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,
  - les copolymères de la vinylpyrrolidone tels que : le copolymère vinylpyrrolidone/1-héxadécène, ANTARON V-216 commercialisé ou fabriqué par la société ISP (MM=7300 g/mol),
- les esters tels que :
  - les esters d'acides gras linéaires ayant un nombre total de carbone allant de 35 à 70 comme le tétrapélargonate de pentaérythrityle (MM=697,05 g/mol),
  - les esters hydroxylés tels que le triisostéarate de polyglycérol-2 (MM=965,58 g/mol),
  - les esters aromatiques tels que le tridécyl trimellitate (MM=757,19 g/mol),
  - les esters d'alcool gras ou d'acides gras ramifiés en $C_{24}$-$C_{28}$ tels que ceux décrits dans la demande EP-A-0 955 039, et notamment le citrate de triisoarachidyle (MM=1033,76 g/mol), le tétraisononanoate de pentaérythrityle (MM=697,05g/mol), le triisostéarate de glycéryle (MM=891.51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
- les huiles siliconées telles que les silicones phénylées comme la BELSIL PDM 1000 de la société WACKER (MM=9000 g/mol),
- les huiles d'origine végétale telles que l'huile de sésame (820,6 g/mol),
- et leurs mélanges.

**<u>Polymère séquencé :</u>**

La composition selon la présente invention contient au moins un polymère séquencé. Par polymère "séquencé", on entend un polymère comprenant au moins 2 séquences distinctes, de préférence au moins 3 séquences distinctes.

Selon un mode de réalisation, le polymère séquencé de la composition selon l'invention est un polymère éthylénique. Par polymère "éthylénique", on entend un polymère obtenu par polymérisation de monomères comprenant une insaturation éthylénique.

Selon un mode de réalisation, le polymère séquencé de la composition selon l'invention est un polymère linéaire. Par opposition, un polymère à structure non linéaire est, par exemple, un polymère à structure ramifiée, en étoile, greffée, ou autre.

Selon un mode de réalisation, le polymère séquencé de la composition selon l'invention est un polymère filmogène. Par polymère "filmogène", on entend un polymère apte à former à lui seul ou en présence d'un agent auxiliaire de filmification, un film continu et adhérent sur un support, notamment sur les matières kératiniques.

Selon un mode de réalisation, le polymère séquencé de la composition selon l'invention est un polymère non élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomère" on désigne un polymère ayant une récouvrance instantanée $R_i$ < à 50% et une recouvrance retardée $R_{2h}$ < 70% après avoir subi un allongement de 30%. De préférence, $R_i$ est < à 30 %, et $R_{2h}$ < 50 %.

Plus précisément, le caractère non élastomère du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.
On obtient alors un film d'environ 100 μm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.
Les éprouvettes sont étirées à une vitesse de 50 mm/min et la distance entre les mors est de 50 mm, ce qui correspond à la longueur initiale ($I_o$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($I_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure l'allongement résiduel de l'éprouvette en pourcentage ($\varepsilon_{2h}$), 2 heures après retour à la contrainte nulle.

La recouvrance retardée en % ($R_{2h}$) est donnée par la formule ci-après:

$$R_{2h} = (\varepsilon_{max} - \varepsilon_{2h})/\varepsilon_{max}) \times 100$$

A titre purement indicatif, un polymère selon un mode de réalisation de l'invention possède une recouvrance instantanée $R_i$ de 10% et une recouvrance retardée $R_{2h}$ de 30%.

Selon un autre mode de réalisation, le polymère séquencé de la composition selon l'invention ne comprend pas de motif styrène. Par polymère exempt de motif styrène, on entend un polymère comprenant moins de 10%, de préférence moins de 5%, de préférence moins de 2%, de préférence encore moins de 1% en poids i) de motif styrène de formule $-CH(C_6H_5)-CH_2-$ ou ii) de motif styrène substitué, comme par exemple le méthylstyrène, le chlorostyrène ou le chlorométhylstyrène.

Selon un mode de réalisation, le polymère séquencé de la composition selon l'invention est issu de monomères éthyléniques aliphatiques. Par monomère aliphatique, on entend un monomère ne comprenant aucun groupe aromatique.

Selon un mode de réalisation, le polymère séquencé est un polymère éthylénique issu de monomères éthyléniques aliphatiques comprenant une double liaison carbone carbone et au moins un groupement ester -COO- ou amide -CON-. Le groupe ester peut être lié à un des deux carbones insaturés par l'atome de carbone ou l'atome d'oxygène. Le groupe amide peut être lié à un des deux carbones insaturés par l'atome de carbone ou l'atome d'azote.
Selon un mode de mise en oeuvre, le polymère séquencé comprend au moins une première séquence et au moins une

deuxième séquence telles que définies précédemment.
Par "au moins" une séquence, on entend une ou plusieurs séquences.
On précise que dans ce qui précède et ce qui suit les termes "première" et "deuxième" séquences ne conditionnent nullement l'ordre desdites séquences (ou blocs) dans la structure du polymère.

De préférence, la séquence intermédiaire est issue essentiellement de monomères constitutifs de la première séquence et de la deuxième séquence.
Par "essentiellement", on entend au moins à 85%, de préférence au moins à 90%, mieux à 95% et encore mieux à 100%.

Selon un mode de mise en oeuvre, le polymère séquencé comprend au moins une première séquence et au moins une deuxième séquence incompatibles dans le milieu liquide organique de la composition de l'invention.
Par "séquences incompatibles l'une avec l'autre", on entend que le mélange formé du polymère correspondant à la première séquence et du polymère correspondant à la deuxième séquence, n'est pas miscible dans le liquide majoritaire en poids contenu dans le milieu liquide organique de la composition, à température ambiante (25°C) et pression atmosphérique ($10^5$ Pa), pour une teneur du mélange de polymères supérieure ou égale à 5 % en poids, par rapport au poids total du mélange (polymères et liquide organique majoritaire), étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé +/- 15%.

Dans le cas où le milieu liquide organique comprend un mélange de liquides organiques, dans l'hypothèse de deux ou plusieurs liquides présents en proportions massiques identiques, ledit mélange de polymères est non miscible dans au moins l'un d'entre eux.

Dans le cas où le milieu liquide organique comprend un seul liquide organique, ce dernier constitue bien évidemment le liquide majoritaire en poids.

Par "milieu liquide organique", on entend un milieu contenant au moins un liquide organique, c'est-à-dire, au moins un composé organique liquide à température ambiante (25°C) et pression atmosphérique ($10^5$ Pa). Selon un mode de mise en oeuvre, le liquide majoritaire du milieu liquide organique est une huile (corps gras) volatile ou non volatile. De préférence, le liquide organique est cosmétiquement acceptable (tolérance, toxicologie et toucher acceptables). Le milieu liquide organique est cosmétiquement acceptable, en ce sens qu'il est compatible avec les matières kératiniques, comme les huiles ou les solvants organiques couramment employés dans les compositions cosmétiques.

Selon un mode de mise en oeuvre, le liquide majoritaire du milieu liquide organique est le solvant ou un des solvants de polymérisation du polymère séquencé tels qu'ils sont décrits ci-après.
Par solvant de polymérisation, on entend un solvant ou un mélange de solvants. Le solvant de polymérisation peut être choisi notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol, ou l'isododécane.

De manière générale, le polymère séquencé peut être incorporé dans la composition à une teneur élevée en matières sèche, typiquement supérieure à 10%, supérieure à 20% et de préférence encore supérieure à 30% et de préférence encore supérieure à 45% en poids par rapport au poids total de la composition tout en étant faciles à formuler.

De façon préférentielle, le polymère séquencé ne comprend pas d'atomes de silicium dans son squelette. Par "squelette", on entend la chaîne principale du polymère, par opposition aux chaînes latérales pendantes.

De préférence, le polymère selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

Avantageusement, le polymère séquencé utilisé dans les compositions selon l'invention a un indice de polydispersité I allant de 2,8 à 6.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère séquencé est de préférence inférieure ou égale à 300 000, elle va par exemple de 35 000 à 200 000, et mieux de 45 000 à 150 000.

La masse moyenne en nombre (Mn) du polymère séquencé est de préférence inférieure ou égale à 70 000, elle va par exemple de 10 000 à 60 000, et mieux de 12 000 à 50 000.

Chaque séquence ou bloc du polymère séquencé est issue d'un type de monomère ou de plusieurs types de

monomères différents.
Cela signifie que chaque séquence peut être constituée d'un homopolymère ou d'un copolymère ; ce copolymère constituant la séquence pouvant être à son tour statistique ou alterné.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_i (\varpi_i / Tg_i),$$

$\omega_i$ étant la fraction massique du monomère i dans la séquence considerée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

<u>a) Séquence avant une Tg supérieure ou égale à 40°C</u>

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C.

La séquence ayant une Tg supérieure ou égale à 40°C peut être un homopolymère ou un copolymère.

La séquence ayant une Tg supérieure ou égale à 40°C peut être issue en totalité ou en partie de un ou plusieurs monomères, qui sont tels que l'homopolymère préparé à partir de ces monomères a une températures de transition vitreuse supérieure ou égale à 40°C.

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20 à 40°C et/ou les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à tels que décrits plus loin.

Les monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
  dans laquelle $R_2$ représente un groupe cycloalkyle en $C_4$ à $C_{12}$ tel que l'acrylate d'isobornyle ou un groupe tertio

butyle,

- les (méth)acrylamides de formule :

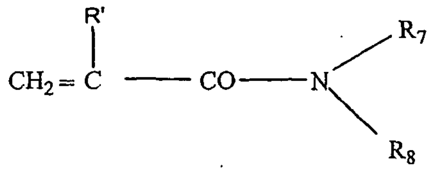

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,

- et leurs mélanges.

Des monomères principaux particulièrement préférés sont le méthacrylate de méthyle, le (méth)acrylate d'isobutyle, le (méth)acrylate d'isobornyle et leurs mélanges.

b) Séquence avant une Tg inférieure ou égale à 20°C

La séquence ayant une Tg inférieure ou égale à 20°C a par exemple une Tg allant de - 100 à 20°C, de préférence inférieure ou égale à 15°C, notamment allant de -80°C à 15°C et mieux inférieure ou égale à 10°C, par exemple allant de -50°C à 0°C.

La séquence ayant une Tg inférieure ou égale à 20°C peut être un homopolymère ou un copolymère.

La séquence ayant une Tg inférieure ou égale à 20°C peut être issue en totalité ou en partie de un ou plusieurs monomères, qui sont tel(s) que homopolymère préparé à partir de ces monomères a une température de transition vitreuse inférieure ou égale à 20°C.

Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).

Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.
Elle peut par exemple comprendre.

- un ou plusieurs monomères dont l'homopolymère correspondant a une Tg inférieure ou égale à 20°C, par exemple une Tg allant de -100°C à 20 °C, de préférence inférieure à 15°C, notamment allant de - 80 °C à 15°C et mieux inférieur à 10°C, par exemple allant de -50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.

Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_s\text{-}CO\text{-}O\text{-}CH = CH_2$
  où $R_5$ représente un groupe alkyle en $C_4$ à $C_{12}$ linéaire ou ramifié ;

- les éthers de vinyle et d'alkyle en $C_4$ à $C_{12}$,

- les N-alkyl en $C_4$ à $C_{12}$ acrylamides, tels que le N-octylacrylamide,

- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

<u>c) Séquence ayant une Tg comprise entre 20 et 40°C</u>

La séquence qui a une Tg comprise entre 20 et 40°C peut être un homopolymère ou un copolymère.
La séquence ayant une Tg comprise entre 20 et 40°C peut être issue en totalité ou en partie de un ou de plusieurs monomères, qui sont tel(s) que l'homopolymère préparé à partir de ces monomères a une température de transition vitreuse comprise entre 20 et 40°C.
La séquence ayant une Tg comprise entre 20 et 40°C peut être issue en totalité ou en partie de monomères qui sont tel(s) que l'homopolymère correspondant a une Tg supérieure ou égale à 40°C et de monomères qui sont tel (s) que l'homopolymère correspondant a une Tg inférieure ou égale à 20°C.
Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40°C. Cette première séquence peut être un homopolymère, constitué par un seul type de monomère (dont la Tg de l'homopolymère correspondant va de 20°C à 40°C).
Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.
Dans le cas où la séquence ayant une Tg comprise entre 20 et 40°C est un copolymère elle est issue en totalité ou en partie de un ou de plusieurs monomères (ou monomères principaux), dont la nature et la concentration sont choisis de telle sorte que la Tg du copolymère résultant soit comprise entre 20 et 40°C.
Avantageusement, la séquence ayant une Tg comprise entre 20 et 40°C est un copolymère issue en totalité ou en partie :

- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et/ou
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C., par exemple allant de -50°C à 0°C, tels que décrits plus haut,

lesdits monomères étant choisis de telle sorte que la Tg du copolymère formant la première séquence est comprise entre 20 et 40°C.
De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.
De préférence, la proportion de la deuxième séquence ayant une Tg inférieure ou égale à 20°C va de 10 à 85% en poids du polymère, mieux de 20 à 70% et encore mieux de 20 à 50%.
De préférence, chacune des première et deuxième séquences comprend au moins un monomère choisi parmi l'acide acrylique, les esters d'acide acrylique, l'acide (méth)acrylique, les esters d'acide (méth)acrylique et leurs mélanges.
Avantageusement, chacune des première et deuxième séquences est issue en totalité d'au moins un monomère choisi parmi l'acide acrylique, les esters d'acide acrylique, l'acide (méth)acrylique, les esters d'acide (méth)acrylique et leurs mélanges.
Chacune des séquences peut néanmoins contenir en proportion minoritaire au moins un monomère constitutif

de l'autre séquence.

Ainsi la première séquence peut contenir au moins un monomère constitutif de la deuxième séquence et inversement.

Chacune des première et/ou deuxième séquence, peu(ven)t comprendre, outre monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

a) les monomères hydrophiles tels que :

- les monomères à insaturation(s) éthylénique(s) comprenant au moins une fonction acide carboxylique ou sulfonique comme par exemple :

  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,

$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;

- les acrylates de formule $CH_2 = CHCOOR_{10}$,

$R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{l2}$)-O-POE (polyoxyéthylène) avec répétition du motif oxyéthylène de 5 à 30 fois, par exemple méthoxy-POE, ou $R_{10}$ représente un groupement polyoxyéthyléné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris ( triméthylsiloxy) silane,

- et leurs mélanges.

Des monomères additionnels particulièrement préférés sont l'acide acrylique, l'acide méthacrylique, le méthacrylate de trifluoroéthyle et leurs mélanges.

Selon un mode de réalisation, chacune des première et deuxième séquence du polymère séquence comprend au moins un monomère choisi parmi les esters d'acide (méth)acrylique et éventuellement au moins un monomère additionnel tel que l'acide (méth)acrylique, et de leurs mélanges.

Selon un autre mode de réalisation, chacune des première et deuxième séquence du polymère séquencé est issue en totalité d'au moins un monomère choisi parmi les esters d'acide (méth)acrylique et éventuellement d'au moins un monomère additionnel tel que l'acide (méth)acrylique, et de leurs mélanges.

Selon un mode préféré de réalisation, le polymère séquencé est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

Le polymère séquencé peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' (allant de 3 à 6 h) au bout duquel le mélange est ramené à température ambiante,
- on obtient le polymère en solution dans le solvant de polymérisation.

Premier mode de réalisation

Selon un premier mode de réalisation, le polymère séquencé comprend une première séquence ayant une Tg supérieure ou égale à 40°C, telle que décrite plus haut au a) et une deuxième séquence ayant une Tg inférieure ou égale à 20°C, telle que décrite plus haut au b).

De préférence, la première séquence ayant une Tg supérieure ou égale à 40°C est un copolymère issu de monomères qui sont tels que l'homopolymère préparé à partir de ces monomères a une température de transition vitreuse supérieure ou égale à 40°C, tels que les monomère décrits plus haut.

Avantageusement, la deuxième séquence ayant une Tg inférieure ou égale à 20°C est un homopolymère issu de monomères qui sont tel(s) que homopolymère préparé à partir de ces monomères a une température de transition vitreuse inférieure ou égale à 20°C, tels que les monomères décrits plus haut.

De préférence, la proportion de la séquence ayant une Tg supérieure ou égale à 40°C va de 20 à 90% en poids du polymère, mieux de 30 à 80% et encore mieux de 50 à 70%.

De préférence, la proportion de la séquence ayant une Tg inférieure ou égale à 20°C va de 5 à 75% en poids du polymère, de préférence de 15 à 50% et mieux de 25 à 45%.

Ainsi, selon une première variante, le polymère selon l'invention peut comprendre :

- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante, le polymère selon invention peut comprendre:

- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et
- une séquence intérmédiaire qui est un copolymère statistique méthacrylate de méthyl/acide acrylique/acrylate de méthyle/méthacrylate de trifluoroéthyle.

Selon une troisième variante, le polymère selon l'invention peut comprendre :

- une première séquence de Tg supérieure ou égale à 40°C, par exemple allant de 85 à 115°C, qui est un copolymère acrylate d'isobornyle/méthacrylate d'isobutyle,
- une deuxième séquence de Tg' inférieure ou égale à 20°C, par exemple allant de -85 à - 55°C, qui est un homopolymère d'acrylate d'éthyl-2 hexyle et
- une séquence intermédiaire qui est un copolymère statistique acrylate d'isobornyle/méthacrytate d'isobutyte/acrytate d'éthyl-2 hexyle.

Selon une quatrième variante, le polymère selon l'invention peut comprendre :

- une première séquence de Tg supérieure ou égale à 40°C, par exemple allant de 85 à 115°C, qui est un copolymère acrylate d'isobornyle/méthacrylate de méthyle,

- une deuxième séquence de Tg inférieure ou égale à 20°C, par exemple allant de -85 à
- 55°C, qui est un homopolymère d'acrylate d'éthyl-2 hexyle et
- une séquence intermédiaire qui est un copolymère statistique acrylate d'isobornyle/méthacrylate de méthyle/acrylate d'éthyl-2 hexyle.

Selon une cinquième variante, le polymère selon l'invention peut comprendre :

- une première séquence de Tg supérieure ou égale à 40°C, par exemple allant de 95 à 125°C, qui est un copolymère acrylate d'isobornyle / méthacrylate d'isobornyle,
- une deuxième séquence de Tg inférieure ou égale à 20°C, par exemple allant de -85 à
- 55°C, qui est un homopolymère d'acrylate d'éthyl-2 hexyle et
- une séquence intermédiaire qui est un copolymère statistique acrylate d'isobornyle/ méthacrylate d'isobornyle/ acrylate d'éthyl-2 hexyle.

Selon une sixième variante, le polymère selon l'invention peut comprendre :

- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à
- 5°C, qui est un homopolymère d'acrylate d'isobutyle et
- une séquence intermédiaire qui est un copolymère statistique méthacrylate d'isobornyle/méthacrylate d'isobutyle/ acrylate d'isobutyle.

Selon une septième variante, le polymère selon l'invention peut comprendre :

- une première séquence de Tg supérieure ou égale à 40°C, par exemple allant de 95 à 125°C, qui est un copolymère acrylate d'isobornyle/méthacrylate d'isobornyle,
- une deuxième séquence de Tg inférieure ou égale à 20°C, par exemple allant de -35 à
- 5°C, qui est un homopolymère d'acrylate d'isobutyle et
- une séquence intermédiaire qui est un copolymère statistique acrylate d'isobornyle/méthacrylate d'isobornyle/acrylate d'isobutyle.

Selon une huitième variante, le polymère selon l'invention peut comprendre :

- une première séquence de Tg supérieure ou égale à 40°C, par exemple allant de 60 à 90°C, qui est un copolymère acrylate d'isobornyle/méthacrylate d'isobutyte,
- une deuxième séquence de Tg inférieure ou égale à 20°C, par exemple allant de -35 à
- 5°C, qui est un homopolymère d'acrylate d'isobutyle et
- une séquence intermédiaire qui est un copolymère statistique acrylate d'isobornyle/méthacrylate d'isobutyle/acrylate d'isobutyle.

Les exemples qui suivent illustrent de manière non limitative des polymères correspondant à ce premier mode de réalisation.
Les quantités sont exprimées en gramme.

**Exemple 1 : Préparation d'un Polymère de poly(méthacrylate de méthyle/acide acrylique/acrylate de méthyle)**

100 g d'acétate de butyle sont introduits dans un réacteur de 1 litre, puis on augmente la température de façon à passer de la température ambiante (25°C) à 90°C en 1 heure. On ajoute ensuite, à 90°C et en 1 heure, 180 g de méthacrylate de méthyle, 30 g d'acide acrylique, 40 g d'acétate de butyle, 70 g d'isopropanol et 1,8 g de 2.5- Bis (2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
On obtient un polymère comprenant une première séquence ou bloc poly (méthacrylate de méthyle/acide

acrylique) ayant une Tg de 100°C une deuxième séquence ou bloc polyacrylate de méthyle ayant une Tg de 10°C et une séquence intermédiaire qui est un polymère statistique méthacrylate de méthyle/acide acrylique/polyacrylate de méthyle.

Ce polymère présente une masse moyenne en poids de 52000 et une masse moyenne en nombre de 18000, soit un indice de polydispersité I de 2,89.

**Exemple 2 : Préparation d'un polymère de Poly(acrylate d'isobornyle/méthacrylate d'isobutyle/acrylate d'éthyl-2 hexyle)**

100 g d'isododécane sont introduits dans un réacteur de 1 litre, puis on augmente la température de façon à passer de la température ambiante (25°C) à 90°C en 1 heure. On ajoute ensuite, à 90°C et en 1 heure, 120 g d'acrylate d'isobornyle, 90 g de méthacrylate d'isobutyle, 110 g d'isododécane et 1,8 g de 2.5- Bis(2-éthylhexanoylperoxy)-2.5-diméthylhexane (Trigonox® 141 d'Akzo Nobel).

Le mélange est maintenu 1 h 30 à 90°C.

On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.

Le mélange est maintenu 3 heures à 90°C, puis l'ensemble est refroidi.

On obtient une solution à 50% de matière active en polymère dans l'isododécane.

On obtient un polymère comprenant une première séquence ou bloc poly(acrylate d'isobornyle/méthacrylate d'isobutyle) ayant une Tg de 80°C, une deuxième séquence polyacrylate d'éthyl-2 hexyle ayant une Tg de - 70°C et une séquence intermédiaire qui est un polymère statistique acrylate d'isobornyle/méthacrylate d'isobutyle/acrylate d'éthyl-2 hexyle.

Ce polymère présente une masse moyenne en poids de 77 000 et une masse moyenne en nombre de 19 000, soit un indice de polydispersité I de 4,05.

**Exemple 3 : Préparation d'un polymère de poly(acrylate d'isobornyle/méthacrylate d'isobornyle/acrylate d'éthyl-2 hexyle)**

100 g d'isododécane sont introduits dans un réacteur de 1 litre, puis on augmente la température dé façon à passer de la température ambiante (25°C) à 90°C en 1 heure.

On ajoute ensuite, à 90°C et en 1 heure, 105 g d'acrylate d'isobornyle, 105 g de méthacrylate d'isobomyl, 110 g d'isododécane et 1,8 g de 2.5- Bis(2-éthylhexanoylperoxy)-2.5-diméthylhexane (Trigonox® 141 d'Akzo Nobel).

Le mélange est maintenu 1 h 30 à 90°C.

On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.

Le mélange est maintenu 3 heures à 90°C, puis l'ensemble est refroidi.

On obtient une solution à 50% de matière active en polymère dans l'isododécane.

On obtient un polymère comprenant une première séquence ou bloc poly(acrylate d'isobornyle/méthacrylate d'isobornyle) ayant une Tg de 110°C, une deuxième séquence polyacrylate d'éthyl-2 hexyle ayant une Tg de - 70°C et une séquence intermédiaire qui est un polymère statistique acrylate d'isobornyle/méthacrylate d'isobornyle/acrylate d'éthyl-2 hexyle.

Ce polymère présente une masse moyenne en poids de 103 900 et une masse moyenne en nombre de 21 300, soit un indice de polydispersité 1 de 4,89.

**Exemple 4: Préparation d'un polymère de poly(acrylate d'isobornyle/methacrylate d'isobutyle /acrylate d'isobutyle)**

100 g d'isododécane sont introduits dans un réacteur de 1 litre, puis on augmente la température de façon à passer de la température ambiante (25°C) à 90°C en 1 heure. On ajoute ensuite, à 90°C et en 1 heure, 120 g de acrylate d'isobornyle, 90 g de méthacrylate d'isobutyle, 110 g d'isododécane et 1,8 g de 2.5- Bis(2-éthylhexanoylperoxy)-2.5-diméthylhexane (Trigonox® 141 d'Akzo Nobel).

Le mélange est maintenu 1 h 30 à 90°C.

On introduit ensuite au mélange précédent, toujours à 90°C. et en 30 minutes, 90 g d'acrylate d'isobutyle, 90 g d'isododécane et 1,2 g de 2.5- Bis(2-éthylhexanoylperoxy)-2.5-diméthylhexane.

Le mélange est maintenu 3 heures à 90°C, puis l'ensemble est refroidi.

On obtient une solution à 50% de matière active en polymère dans l'isododécane.

On obtient un polymère comprenant une première séquence ou bloc poly(acrylate d'isobornyle/méthacrylate d'isobutyle) ayant une Tg de 75°C, une deuxième séquence polyacrylate d'isobutyle ayant une Tg de - 20°C et une

séquence intermédiaire qui est un polymère statistique acrylate d'isobornyle/méthacrylate d'isobutyle/acrylate d'isobutyle.

**Exemple 5: Préparation d'un polymère de poly(méthacrylate de méthyle/acrylate de méthyl/acide acrylique)**

100 g d'acétate de butyle sont introduits dans un réacteur de 1 litre, puis on augmente la température de façon à passer de la température ambiante (25°C) à 90°C en 1 heure.
On ajoute ensuite, à 90°C et en 1 heure, 50,4 g de méthacrylate de méthyle, 21 g d'acide acrylique, 138,6 g d'acrylate de méthyle, 40 g d'acétate de butyle, 70 g d'isopropanol et 1,8 g de 2.5-Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g de méthacrylate de méthyle, 70 g d'acétate de butyle, 20 g d'isopropanol et 1,2 g de 2.5-Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
Le polymère obtenu comprend une première séquence ou bloc poly(acrylate de méthyle/méthacrylate de méthyle/acide acrylique) ayant une Tg de 35°C une deuxième séquence ou bloc poly (méthacrylate de méthyle) ayant une Tg de 100°C et une séquence intermédiaire qui est un polymère statistique méthacrylate de méthyle/acide acrylique/polyacrylate de méthyle.

**Exemple 6 : Préparation d'un polymère de poly(acrylate d'isobornyle/méthacrytate d'isobornyle /acrylate d'isobutyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 105 g d'acrylate d'isobornyle, 105 g de méthacrylate d'isobornyl, 110 g d'isododécane et 1,8 g de 2.5- Bis(2-éthythexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.
On obtient un polymère comprenant une première séquence ou bloc poly(acrylate d'isobornyle/méthacrylate d'isobornyle) ayant une Tg de 110°C, une deuxième séquence polyacrylate d'isobutyle ayant une Tg de - 20°C et une séquence intermédiaire qui est un polymère statistique acrylate d'isobornyle/méthacrylate d'isobornyle/acrylate d'isobutyle.
Ce polymère présente une masse moyenne en poids de 151 000 et une masse moyenne en nombre de 41 200, soit un indice de polydispersité 1 de 3.66.

**Exemple 7 : Préparation d'un polymère de poly(methacrylate d'isobornyle/methacrylate d'isobutyle /acrylate d'isobutyle)**

100 g d'isododécane sont introduits dans un réacteur de 1 litre, puis on augmente la température de façon à passer de la température ambiante (25°C) à 90°C en 1 heure. On ajoute ensuite, à 90°C et en 1 heure, 120 g de methacrylate d'isobornyle, 90 g de méthacrylate d'isobutyle, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.
On obtient un polymère comprenant une première séquence ou bloc poly(methacrylate d'isobornyle/méthacrylate d'isobutyle) ayant une Tg de 95°C, une deuxième séquence polyacrylate d'isobutyle ayant une Tg de - 20°C et une séquence intermédiaire qui est un polymère statistique methacrylate d'isobornyle/méthacrylate d'isobutyle/acrylate d'isobutyle.
Ce polymère présente une masse moyenne en poids de 100 700 et une masse moyenne en nombre de 20 800, soit un indice de polydispersité I de 4.85.

Second mode de réalisation

Selon un second mode de réalisation, le polymère séquencé comprend une première séquence ayant une température de transition vitreuse (Tg) comprise entre 20 et 40°C, conforme aux séquences décrites au c) et une deuxième séquence ayant une température de transition vitreuse inférieure ou égale à 20°C, telle que décrite plus haut au b) ou une température de transition vitreuse supérieure ou égale à 40°C, telle que décrite au a) ci-dessus.
De préférence, la proportion de la première séquence ayant une Tg comprise entre 20 et 40°C va de 10 à 85% en poids du polymère, mieux de 30 à 80% et encore mieux de 50 à 70%.
Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.
Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.
De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.
Avantageusement, la deuxième séquence ayant une Tg inférieure ou égale à 20°C ou ayant une Tg supérieure ou égale à 40°C est un homopolymère.
Ainsi, selon première variante de ce second mode de réalisation, le polymère séquence peut comprendre :

- une première séquence de Tg comprise entre 20 et 40°C, par exemple ayant une Tg de 25 à 39°C, qui est un copolymère comprenant au moins un monomère acrylate de méthyle, au moins un monomère méthacrylate de méthyle et au moins un monomère acide acrylique,
- une deuxième séquence de Tg supérieure ou égale à 40°C, par exemple allant de 85 à 125°C, qui est un homopolymère composé de monomères méthacrylate de méthyle et
- une séquence intermédiaire comprenant au moins un monomère acrylate de méthyle, méthacrylate de méthyle et
- une séquence intermédiaire comprenant au méthacrylate de méthyle, au moins un monomère acide acrylique et au moins un monomère acrylate de méthyle.

Selon seconde variante de ce second mode de réalisation, le polymère séquencé peut comprendre :

- une première séquence de Tg comprise entre 20 et 40°C, par exemple ayant une Tg de 21 à 39°C, qui est un copolymère comprenant acrylate d'isobornyle/méthacrylate d'isobutyle/acrylate d'éthyl-2 hexyle,
- une deuxième séquence de Tg inférieure ou égale à 20°C, par exemple allant de -65 à - 35°C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une troisième variante de ce second mode de réalisation, le polymère séquencé peut comprendre :

- une première séquence de Tg comprise entre 20 et 40°C, par exemple ayant une Tg de 21 à 39°C, qui est un copolymère acrylate d'isobornyle/acrylate de méthyle/acide acrylique,
- une deuxième séquence de Tg supérieure ou égale à 40°C, par exemple allant de 85 à 115°C, qui est un homopolymère d'acrylate d'isobornyle et
- une séquence intermédiaire qui est un copolymère statistique acrylate d'isobornyle / acrylate de méthyle/acide acrylique.

La composition de l'invention contient avantageusement de 0,1 à 60% en poids en matière active (ou matière séche) de polymère séquencé, de préférence de 0,5 à 50 % en poids, et de préférence encore de 1 à 40% en poids.
La composition selon l'invention peut comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carboné comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en Cr$C_4$ hydrophiles.
L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.
La composition selon l'invention comprend un milieu liquide organique cosmétiquement acceptable (tolérance,

toxicologie et toucher acceptables).

Selon un mode de réalisation particulièrement préféré, le milieu liquide organique de la composition contient au moins un solvant organique, qui est le ou un des solvants de polymérisation du polymère séquencé tel que décrit précédemment. Avantageusement, ledit solvant organique est le liquide majoritaire en poids dans le milieu liquide organique de la composition cosmétique.

Selon un mode de mise en oeuvre, le milieu liquide organique comprend au moins un corps gras liquide à température ambiante (25°C en général). Ce corps gras liquide peut être d'origine animale, végétale, minérale ou synthétique.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

Le milieu liquide organique de la composition selon l'invention peut également comprendre un ou plusieurs solvants organiques, cosmétiquement acceptables (tolérance, toxicologie et toucher acceptables).

Ces solvants peuvent être généralement présents en une teneur allant de 0,1 à 90%, de préférence encore de 10 à 90% en poids, par rapport au poids total de la composition, et mieux de 30 à 90 %.

Comme solvants utilisables dans la composition de l'invention, on peut citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène, glycol, l'acétate de monométhyl éther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ; les éthers liquides à température ambiante tels que le diéthyléther, le diméthyléther ou le dichlorodiéthyléther ; les alcanes liquides à température ambiante tels que le décane, l'heptane, le dodécane, l'isododécane, le cyclohexane ; les composés cycliques aromatiques liquides à température ambiante tels que le toluène et le xylène ; les aldéhydes liquides à température ambiante tels que le benzaldéhyde, l'acétaldéhyde et leurs mélanges.

La composition peut comprendre, outre le polymère séquencé décrit précédemment, polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut comprendre au moins une cire. Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

Le point de fusion de la cire peut être mesuré à l'aide d'un calorimètre à, balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

La nature et la quantité des corps gras solides sont fonction des propriétés mécaniques et des textures recherchées. A titre indicatif, la composition peut contenir de 0 à 50 % en poids de cires, par rapport au poids total de la composition et mieux de 1 à 30 % en poids.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte souple anhydre.

L'homme du métier pourra choisir la forme galénique appropriée, ainsi que sa méthode de préparation, sur la

base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage comme les produits pour le teint (fonds de teint), les fards à joues ou à paupières, les rouges à lèvres liquides, les produits anti-cernes, les mascaras, les eye-liners, les produits de maquillage des sourcils, les produits pour les ongles, tels que les vernis à ongles, les produits de maquillage du corps, les produits de maquillage des cheveux (mascara ou laque pour cheveux).
La composition selon l'invention peut également être un produit de soin de la peau du corps et du visage, notamment un produit solaire ou de coloration de la peau (tel qu'un autobronzant).

La présente invention a également pour objet un ensemble cosmétique comprenant :

- un récipient délimitant au moins un compartiment, ledit récipient étant fermé par un élément de fermeture ; et
- une composition telle que décrite précédemment disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).
Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur

(aérosol). Dans ce dernier cas, le récipient est équipé d'une valve (du type de celles utilisées pour les aérosols).

Le contenu des brevets ou demandes de brevets cités précédemment sont incorporés par référence dans la présente demande.

Les exemples qui suivent illustrent de manière non limitative les compositions selon l'invention.

**Exemple 8 à 12: Rouges à lèvres**

| ***Exemple*** | ***8*** | ***9*** | ***10*** | ***11*** | ***12*** |
|---|---|---|---|---|---|
| Polymère de l'exemple 2 | 90,7 | | | | |
| Polymère de l'exemple 3 | | 90,7 | | | |
| Polymère de l'exemple 4 | | | 90,7 | | |
| Polymère de l'exemple 6 | | | | 90,7 | |
| Polymère de l'exemple 7 | | | | | 90,7 |
| Polyisobutène Hydrogéné | 2,1 | 2,1 | 2,1 | 2,1 | 2,1 |
| Octyldodécanol | 0,9 | 0,9 | 0,9 | 0,9 | 0,9 |
| Phényltriméthicone (DC 556, 20 cSt, Dow Corning) | 2,1 | 2,1 | 2,1 | 2,1 | 2,1 |
| Copolymère vinylpyrrolidone/1-éicosène (Antaron V-220, ISP) | 1,2 | 1,2 | 1,2 | 1,2 | 1,2 |
| pigments | 3 | 3 | 3 | 3 | 3 |

Mode opératoire

1. On réalise un broyat pigmentaire des pigments dans la phase huileuse en effectuant 3 passages du mélange à la broyeuse tri cylindres.
2. On pèse dans un bêcher le broyat nécessaire à la composition et les autres ingrédients.
3. On place le mélange sous agitation Rayneri pendant 45 min à température ambiante.
4. On coule la formule dans des bouillottes étanches à l'isododécane.

Mesure de brillance

1. A l'aide d'un applicateur mécanique on réalise, pour chaque composition, un film dont l'épaisseur humide est de 50 µm et un film dont l'épaisseur humide est de 150 µm. Les dépôts sont réalisés sur une carte de contraste de marque LENETA et de référence FORM 1A PENOPAC.

2. On laisse sécher 24 heures à une température régulée de 30°C.

3. On réalise les mesures de brillance au brillancemètre micro-tri-gloss BYK GARDNER à un angle de mesure de 20° et à un angle de mesure de 60° pour chacune des deux séries de dépôts.

Les résultats de brillance in vitro obtenus figurent dans le tableau ci-dessous :

Dépôt 150µm

| angle | dépôt | Exemple | moyenne (%) | écart type |
|---|---|---|---|---|
| angle de 20° | dépôt 150µm | exemple 8 | 54.2 | 2.0 |
| | | exemple 9 | 41.0 | 3.5 |
| angle de 60° | dépôt 150µm | exemple 8 | 75.7 | 0.8 |
| | | exemple 9 | 73.6 | 1.6 |

Dépôt 50 µm

| angle | dépôt | Exemple | moyenne (%) | écart type |
|---|---|---|---|---|
| angle de 20° | dépôt 50µm | exemple 8 | 47.6 | 1.2 |
| | | exemple 9 | 42.6 | 5.2 |
| angle de 60° | dépôt 50µm | exemple 8 | 69.3 | 0.7 |
| | | exemple 9 | 74.8 | 1.0 |

La brillance moyenne obtenue est supérieure à 40 sur 100 pour un angle de mesure de 20° et une épaisseur de 50µm ou 150µm.

La brillance moyenne obtenue est supérieure à 65 sur 100 pour un angle de mesure de 60° et une épaisseur de 50µm. La brillance moyenne obtenue est supérieure à 70 sur 100 pour un angle de mesure de 60° et une épaisseur de 150 µm.

**Exemple 13 : Vernis à ongles**

| | |
|---|---|
| Polymère de l'exemple 1 | 23,8 g en MA |
| Acétate de Butyle | 24,99 g |
| Isopropanol | 10,71 g |
| Hexylène Glycol | 2,5 g |
| DC RED 7 Lake | 1 g |
| Hectorite modifiée par du chlorure de stéaryl di méthyl benzyl ammonium (Bentone® 27V d'Elementis) | 1,3 g |

**Exemple 14 : Vernis à ongles**

| | |
|---|---|
| Polymère de l'exemple 5 | 23,8 g en MA |
| Acétate de Butyle | 24,99 g |
| Isopropanol | 10,71 g |
| Hexylène Glycol | 2,5 g |

(suite)

| | |
|---|---|
| DC RED 7 Lake | 1 g |
| Hectorite modifiée par du chlorure de stéaryl di méthyl benzyl ammonium (Bentone® 27V d'Elementis) | 1,3 g |
| Acétate d'éthyle | qsp 100 g |

**Revendications**

1. Composition cosmétique liquide contenant un milieu liquide organique cosmétiquement acceptable, et un polymère éthylénique séquencé linéaire filmogène non élastomère,
   ledit polymère séquencé contenant des première et deuxième séquences reliées entre elles par un segment intermédiaire qui est un polymère statistique comprenant au moins un monomère constitutif de la première séquence et au moins un monomère constitutif de la deuxième séquence,
   la première séquence du polymère étant choisie parmi :

   - a) une séquence ayant une Tg supérieure ou égale à 40°C,
   - b) une séquence ayant une Tg inférieure ou égale à 20°C,
   - c) une séquence ayant une Tg comprise entre 20 et 40°C, et

   la deuxième séquence étant choisie dans une catégorie a), b) ou c) différente de la première séquence ;
   et ledit polymère séquencé ayant un indice de polydispersité I supérieur à 2,8,
   ledit polymère étant tel que, lorsqu'il est en quantité suffisante dans la composition, la brillance moyenne à 20° d'un dépôt de ladite composition, une fois étalée sur un support, est supérieure ou égale à 30 sur 100.

2. Composition cosmétique liquide contenant un milieu liquide organique cosmétiquement acceptable, et un polymère éthylénique séquencé linéaire filmogène exempt de motif styrène,
   ledit polymère séquencé contenant des première et deuxième séquences reliées entre elles par un segment intermédiaire qui est un polymère statistique comprenant au moins un monomère constitutif de la première séquence et au moins un monomère constitutif de la deuxième séquence,
   la première séquence du polymère étant choisie parmi :

   - a) une séquence ayant une Tg supérieure ou égale à 40°C,
   - b) une séquence ayant une Tg inférieure ou égale à 20°C,
   - c) une séquence ayant une Tg comprise entre 20 et 40°C, et

   la deuxième séquence étant choisie dans une catégorie a), b) ou c) différente de la première séquence :
   et ledit polymère séquence ayant un indice de polydispersité I supérieur à 2,8.
   le polymère étant tel que, lorsqu'il est en quantité suffisante dans la composition, la brillance moyenne à 20° d'un dépôt de ladite composition, une fois étalée sur un support, est supérieure ou égale à 30 sur 100.

3. Composition cosmétique selon la revendication 1 ou 2, **caractérisée en ce que** le polymère séquencé est un polymère éthylénique issu de monomères éthyléniques aliphatiques comprenant une double liaison carbone carbone et au moins un groupement ester -COO- ou amide -CON-.

4. Composition cosmétique selon l'une des revendications précédentes, **caractérisé en ce que** le polymère n'est pas soluble à une teneur en matière active d'au moins 1% en poids dans l'eau ou dans un mélange d'eau et de monoalcools inférieures linéaires ou ramifiés ayant de 2 à 5 atomes de carbone, sans modification de pH, à température ambiante (25°C).

5. Composition selon la revendication précédente, **caractérisée en ce que** les première et deuxième séquences sont reliées entre elles par un segment intermédiaire ayant une température de transition vitreuse comprise entre les températures de transition vitreuse des première et deuxième séquences.

6. Composition cosmétique selon l'une quelconque des revendications précédentes, **caractérisée en ce que** le polymère séquencé contient des première et deuxième séquences incompatibles dans ledit milieu liquide organique.

**7.** Composition selon l'une quelconque des revendications précédentes, **caractérisée en ce que** la séquence ayant une Tg supérieure ou égale à 40°C est issue en totalité ou en partie de un ou plusieurs monomères, qui sont tels que l'homopolymère préparé à partir de ces monomères a une température de transition vitreuse supérieure ou égale à 40°C.

**8.** Composition selon la revendication précédente, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

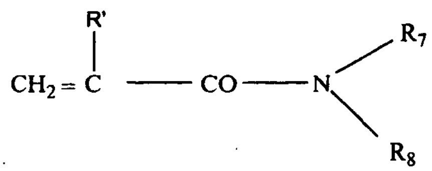

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle.
- et leurs mélanges.

**9.** Composition selon la revendication 7 ou 9, **caractérisée en ce que** les monomères dont l'homopolymère correspondant a une température de transition vitreuse supérieure ou égale à 40°C sont choisis parmi le méthacrylate de méthyle, le (méth)acrylate d'isobutyle, le (méth)acrylate d'isobornyle et leurs mélanges.

**10.** Composition selon l'une quelconque des revendications 1 à 6, **caractérisée en ce que** la séquence ayant une Tg inférieure ou égale à 20°C est issue en totalité ou en partie de un ou plusieurs monomères, qui sont tel(s) que l'homopolymère préparé à partir de ces monomères a une température de transition vitreuse inférieure ou égale à 20°C.

**11.** Composition selon la revendication précédente, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-}CO\text{-}O\text{-}CH = CH_2$ où $R_5$ représente un groupe alkyle en $C_4$ à $C_{12}$ linéaire ou ramifié ;
- les éthers de vinyle et d'alkyle en $C_4$ à $C_{12}$,
- les N-alkyl en $C_4$ à $C_{12}$ acrylamides, tels que le N-octylacrylamide,
- et leurs mélanges.

**12.** Composition selon la revendication 10 ou 11, **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

**13.** Composition selon l'une quelconques des revendications 1 à 6, **caractérisée en ce que** la séquence ayant une Tg comprise entre 20 et 40°C est issue en totalité ou en partie de un ou de plusieurs monomères, qui sont tel(s) que l'homopolymère préparés à partir de ces monomères a une température de transition vitreuse comprise entre 20 et 40°C.

**14.** Composition selon la revendication précédente, **caractérisée en ce que** la séquence ayant une Tg comprise entre 20 et 40°C est issue en totalité ou en partie de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**15.** Composition selon la revendication 13 ou 14, **caractérisée en ce que** la séquence ayant une Tg comprise entre 20 et 40°C est issue en totalité ou en partie de monomères choisis parmi le méthacrylate de méthyle, l'acrylate et le méthacrylate d'isobornyle, l'acrylate de butyle, l'acrylate d'éthyl-2 hexyle et leurs mélangés.

**16.** Composition selon l'une quelconque des revendications 1 à 12, **caractérisée en ce qu'**elle comprend un polymère séquencé comprenant au moins une première séquence et au moins une deuxième séquence, la première séquence ayant une température de transition vitreuse (Tg) supérieure ou égale à 40°C et la deuxième séquence ayant une température de transition vitreuse inférieure ou égale à 20°C.

**17.** Composition selon la revendication précédente, **caractérisée en ce que** la première séquence est issue en totalité ou en partie de un ou plusieurs monomères, qui sont tels que l'homopolymère préparé à partir de ces monomères a une température de transition vitreuse supérieure ou égale à 40°C.

**18.** Composition selon la revendication précédente, **caractérisée en ce que** la première séquence est un copolymère issu de monomères qui sont tels que l'homopolymère préparé à partir de ces monomères a une température de transition vitreuse supérieure ou égale à 40°C.

**19.** Composition selon la revendication 17 ou 18, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

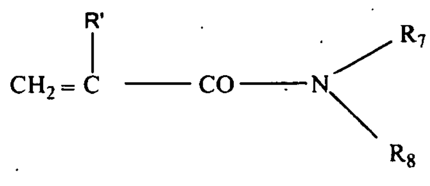

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle,
- et leurs mélanges.

**20.** Composition selon l'une des revendications 17 à 19, **caractérisée en ce que** les monomères dont l'homopolymère

correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

**21.** Composition selon l'une des revendications 16 à 19, **caractérisée en ce que** la proportion de la première séquence va de 20 à 90% en poids du polymère, mieux de 30 à 80% et encore mieux de 50 à 70%.

**22.** Composition selon l'une des revendications 16 à 21, **caractérisée en ce que** la deuxième séquence est issue en totalité ou en partie de un ou plusieurs monomères, qui sont tel(s) que l'homopolymère préparé à partir de ces monomères a une température de transition vitreuse inférieure ou égale à 20°C.

**23.** Composition selon l'une des revendications à 16 à 22, **caractérisée en ce que** la deuxième séquence est un homopolymère issu de monomères qui sont tel(s) que l'homopolymère préparé à partir de ces monomères a une température de transition vitreuse inférieure ou égale à 20°C.

**24.** Composition selon la revendication 22 ou 23, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0. N et S;
- les esters de vinyle de formule $R_5\text{-CO-O-CH} = CH_2$
où $R_5$ représente un groupe alkyle en $C_4$ à $C_{12}$ linéaire ou ramifié ;
- les éthers de vinyle et d'alkyle en $C_4$ à $C_{12}$,
- les N-alkyl en $C_4$ à $C_{12}$ acrylamides, tels que le N-octylacrylamide.
- et leurs mélanges.

**25.** Composition selon l'une des revendications 22 à 24, **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

**26.** Composition selon l'une des revendications 16 à 25, **caractérisée en ce que** la proportion de la deuxième séquence ayant une Tg inférieure ou égale à 20°C va de 5 à 75% en poids du polymère, mieux de 15 à 50% et encore mieux de 25 à 45%.

**27.** Composition selon l'une des revendications 1 à 12, **caractérisée en ce qu'**elle comprend un polymère séquencé comprenant au moins une première séquence et au moins une deuxième séquence, la première séquence ayant une température de transition vitreuse (Tg) comprise entre 20 et 40°C et la deuxième séquence ayant une température de transition vitreuse inférieure ou égale à 20°C ou une température de transition vitreuse supérieure ou égale à 40°C.

**28.** Composition selon la revendication précédente, **caractérisée en ce que** la première séquence ayant une Tg comprise entre 20 et 40°C est issue en totalité ou en partie de un ou de plusieurs monomères, qui sont tel(s) que l'homopolymère préparé à partir de ces monomères a une température de transition vitreuse comprise entre 20 et 40°C.

**29.** Composition selon la revendication 27 ou 28, **caractérisée en ce que** la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**30.** Composition selon lune des revendications 27 à 29, **caractérisée en ce que** la première séquence ayant une Tg comprise entre 20 et 40°C est issue de monomères choisis parmi le méthacrylate de méthyle, l'acrylate et le méthacrylate d'isobornyle, l'acrylate de butyle, l'acrylate d'éthyl-2 hexyle et leurs mélanges.

**31.** Composition selon l'une des revendications 27 à 30, **caractérisée en ce que** la proportion de la première séquence ayant une Tg comprise entre 20 et 40°C va de 10 à 85% en poids du polymère, mieux de 30 à 80% et encore mieux de 50 à 70%.

**32.** Composition selon l'une quelconque des revendications 27 à 31, **caractérisée en ce que** la deuxième séquence a une Tg supérieure ou égale à 40°C et est issue en totalité ou en partie de un ou plusieurs monomères, qui sont tels que l'homopolymère préparé à partir de ces monomères a une température de transition vitreuse supérieure ou égale à 40°C.

**33.** Composition selon l'une quelconque des revendications 27 à 32, **caractérisée en ce que** la deuxième séquence a une Tg supérieure ou égale à 40°C et est un homopolymère issu de monomères, qui sont tels que l'homopolymère préparé à partir de ces monomères a une température de transition vitreuse supérieure ou égale à 40°C.

**34.** Composition selon l'une des revendications 32 ou 33, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

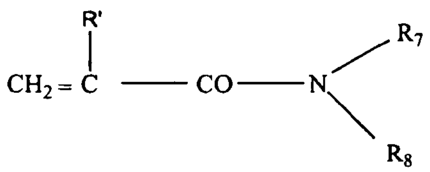

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle,
- et leurs mélanges.

**35.** Composition selon l'une des revendications 32 à 35, **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

**36.** Composition selon l'une des revendications 32 à 35, **caractérisée en ce que** la proportion de la deuxième séquence ayant une Tg supérieure ou égale à 40°C va de 10 à 85%, de préférence de 20 à 70% et mieux de 30 à 70% en poids du polymère.

**37.** Composition selon l'une des revendications 27 à 31, **caractérisée en ce que** la deuxième séquence a une Tg inférieure ou égale à 20°C et est issue en totalité ou en partie de un ou plusieurs monomères, qui sont tel(s) que l'homopolymère préparé à partir de ces monomères a une température de transition vitreuse inférieure ou égale à 20°C.

**38.** Composition selon l'une des revendications 27 à 31, **caractérisée en ce que** la deuxième séquence a une Tg inférieure ou égale à 20°C et est un homopolymère issu de monomères qui sont tel(s) que l'homopolymère préparé à partir de ces monomères a une température de transition vitreuse inférieure ou égale à 20°C

**39.** Composition selon la revendication 37 ou 38, **caractérisée en ce que** les monomères dont l'homopolymère correspondant a une température de transition vitreuse inférieure ou égale à 20°C sont choisis parmi les monomères

suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)-COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5-CO-O-CH = CH_2$ où $R_5$ représente un groupe alkyle en $C_4$ à $C_{12}$ linéaire ou ramifié ;
- les éthers de vinyle et d'alkyle en $C_4$ à $C_{12}$,
- les N-alkyl en $C_4$ à $C_{12}$ acrylamides, tels que le N-octylacrylamide,
- et leurs mélanges.

**40.** Composition selon l'une des revendications 37 à 39, **caractérisée en ce que** les monomères dont les homopolymères ont des températures de transition vitreuse inférieures ou égales à 20°C sont choisis parmi les acrylates d'alkyle dont la chaîne alkyle comprend de 1 à 10 atomes de carbone, à l'exception du groupe tertiobutyle.

**41.** Composition selon l'une des revendications 37 à 40, **caractérisée en ce que** la proportion de la séquence ayant une température de transition vitreuse inférieure ou égale à 20°C, va de 10 à 85% en poids du polymère, mieux de 20 à 70% et encore mieux de 20 à 50%.

**42.** Composition cosmétique selon l'une quelconque des revendications précédentes, **caractérisée en ce que** la première séquence et/ou la deuxième séquence comprend au moins un monomère additionnel.

**43.** Composition selon la revendication précédente, **caractérisée en ce que** le monomère additionnel est choisi parmi les monomères hydrophiles, les monomères à insaturation éthylénique comprenant un ou plusieurs atomes de silicium et leurs mélanges.

**44.** Composition selon la revendication 42 ou 43, **caractérisée en ce que** le monomère additionnel est choisi parmi :

a) les monomères hydrophiles tels que :

- les monomères à insaturation(s) éthylénique(s) comprenant au moins une fonction acide carboxylique ou sulfonique comme par exemple :

l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)-COOR_6$
dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)-COOR_9$,

$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;

- les acrylates de formule $CH_2 = CHCOOR_{10}$,

$R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I est F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène, et

b) les monomères à insaturation éthylénique comprenant un ou plusieurs atomes de silicium tels que le méthacryloxypropyl triméthoxy silane, le méthacryloxypropyl tris ( triméthylsiloxy) silane,

- et leurs mélanges.

**45.** Composition selon l'une des revendications 42 ou 43, **caractérisée en ce que** chacune des première et deuxième séquence comprend au moins un monomère additionnel choisi parmi l'acide acrylique, l'acide (méth)acrylique, le méthacrylate de trifluoroéthyle et leurs mélanges.

**46.** Composition selon l'une des revendications 42 ou 43, **caractérisée en ce que** chacune des première et deuxième séquence comprend au moins un monomère choisi parmi les esters d'acide (méth)acrylique et éventuellement au moins un monomère additionnel tel que l'acide (méth)acrylique, et de leurs mélanges.

**47.** Composition selon l'une des revendications 42 ou 43, **caractérisée en ce que** chacune des première et deuxième séquence est issue en totalité d'au moins un monomère choisi parmi les esters d'acide (méth)acrylique et éventuellement d'au moins un monomère additionnel tel que l'acide (méth)acrylique, et de leurs mélanges.

**48.** Composition selon l'une des revendications 42 à 47, **caractérisée en ce que** le ou les monomères additionnel(s) représente(nt) de 1 à 30% en poids du poids total des première et/ou deuxième séquences.

**49.** Composition selon l'une quelconque des revendications précédentes, **caractérisée en ce que** l'écart entre les températures de transition vitreuse (Tg) des première et deuxième séquences est supérieur à 10°C, mieux, supérieur à 20°C, de préférence supérieure à 30°C et mieux supérieure à 40°C.

**50.** Composition selon l'une quelconque des revendications précédentes, **caractérisée en ce qu'**il a un indice de polydispersité compris entre 2,8 et 6.

**51.** Composition selon l'une des revendications précédentes, **caractérisée en ce que** le polymère séquencé a une masse moyenne en poids (Mw) est inférieure ou égale à 300 000.

**52.** Composition selon la revendication précédente, **caractérisée en ce que** la masse moyenne en poids (Mw) va de 35 000 à 200 000, et mieux de 45 000 à 150 000.

**53.** Composition selon la revendication précédente, **caractérisée en ce que** la masse moyenne en nombre (Mn) est inférieure ou égale à 70 000.

**54.** Composition selon l'une des revendications 51 à 53, dont la masse moyenne en nombre (Mn) va de 10 000 à 60 000, et mieux de 12 000 à 50 000.

**55.** Composition selon l'une des revendications précédentes, **caractérisée en ce que** la brillance moyenne de la composition mesurée à 20° est supérieure ou égale à 30, mieux supérieure ou égale à 35, mieux encore supérieure ou égale à 40, mieux encore supérieure ou égale à 45, mieux encore supérieure ou égale à 50 sur 100, mieux encore, supérieure ou égale à 55, mieux encore, supérieure ou égale à 60, mieux encore, supérieure ou égale à 65, mieux encore, supérieure ou égale à 70 ou mieux encore, supérieure ou égale à 75 sur 100.

**56.** Composition selon l'une des revendications précédentes, **caractérisée en ce que** la brillance moyenne de la composition, une fois étalée sur un support, mesurée à 60° est supérieure ou égale à 50, mieux encore, supérieure ou égale à 60, mieux encore, supérieure ou égale à 65, mieux encore, supérieure ou égale à 70, mieux encore, supérieure ou égale à 75, mieux encore, supérieure ou égale à 80, mieux encore, supérieure ou égale à 85 ou mieux encore, supérieure ou égale à 90 sur 100.

**57.** Composition selon l'une des revendications précédentes, **caractérisée en ce que** la brillance moyenne de la composition mesurée à 20° est supérieure ou égale à 35, de préférence 40, 45 ou 50 sur 100, et/ou la brillance de la composition mesurée à 60° est supérieure ou égale à 65, 70 ou 75 sur 100.

**58.** Composition selon l'une des revendications précédentes, **caractérisée en ce que** la brillance de la composition mesurée à 20° est supérieure ou égale à 60, de préférence 65, 70 ou 75 sur 100, et/ou la brillance de la composition mesurée à 60° est supérieure ou égale à 80, 85 ou 90 sur 100.

**59.** Composition selon l'une des revendications précédentes, **caractérisée en ce qu'**elle comprend de 0,1 à 60 % en poids en matière active de polymère, de préférence de 5 % à 50% en poids, et de préférence encore de 10 à 40 % en poids.

**60.** Composition cosmétique selon l'une quelconque des revendications précédentes **caractérisée en ce qu'**elle comprend, en outre, une ou des matières colorantes choisies parmi les colorants hydrosolubles et les matières colorantes pulvérulentes, tels que les pigments, les nacres et les paillettes.

**61.** Composition cosmétique selon l'une quelconque des revendications précédentes, **caractérisée en ce qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de dispersion de vésicules notamment de lipides ioniques ou non, de lotion biphase ou multiphase, de pâte, notamment de pâte souple ou de pâte anhydre.

**62.** Composition cosmétique selon l'une quelconque des revendications précédentes, **caractérisée en ce qu'**elle se présente sous forme anhydre.

**63.** Composition cosmétique selon l'une quelconque des revendications précédentes, **caractérisée en ce qu'**il s'agit d'une composition de maquillage ou de soin des matières kératiniques.

**64.** Composition cosmétique selon l'une des revendications précédentes, **caractérisée en ce qu'**il s'agit d'un produit de maquillage des lèvres.

**65.** Composition cosmétique selon l'une des revendications précédentes, **caractérisée en ce qu'**il s'agit d'un produit de maquillage des yeux.

**66.** Composition cosmétique selon l'une des revendications précédentes, **caractérisée en ce qu'**il s'agit d'un produit de maquillage des ongles.

**67.** Ensemble cosmétique comprenant :

a) un récipient délimitant au moins un compartiment, ledit récipient étant fermé par un élément de fermeture ; et
b) une composition disposée à l'intérieur dudit compartiment, la composition étant conforme à l'une quelconque des revendications qui précèdent.

**68.** Ensemble cosmétique selon la revendication précédente, **caractérisé en ce que** le récipient est formé, au moins pour partie, en au moins un matériau thermoplastique.

**69.** Ensemble cosmétique selon la revendication 67, **caractérisé en ce que** le récipient est formé, au moins pour partie, en au moins un matériau non thermoplastique, notamment en verre ou en métal.

**70.** Ensemble selon l'une quelconque des revendications 67 à 69, **caractérisé en ce que**, en position fermée du récipient, l'élément de fermeture est vissé sur le récipient.

**71.** Ensemble selon l'une quelconque des revendications 67 à 69, **caractérisé en ce que**, en position fermée du récipient, l'élément de fermeture est couplé au récipient autrement que par vissage, notamment par encliquetage, collage, ou soudage.

**72.** Ensemble selon l'une quelconque des revendications 67 à 71, **caractérisé en ce que** la composition est sensiblement à la pression atmosphérique à l'intérieur du compartiment.

**73.** Ensemble selon l'une quelconque des revendications 67 à 71, **caractérisé en ce que** la composition est pressurisée à l'intérieur du récipient.

**74.** Procédé cosmétique de maquillage ou de soin des matières kératiniques comprenant l'application sur les matières kératiniques d'une composition cosmétique selon l'une des revendications 1 à 66.

## Claims

**1.** Liquid cosmetic composition containing a cosmetically acceptable organic liquid medium and a non-elastomeric film-forming linear block ethylenic polymer,
the said block polymer containing first and second blocks linked together via an intermediate segment which is a random polymer comprising at least one constituent monomer of the first block and at least one constituent monomer of the second block,
the first block of the polymer being chosen from:

- a) a block with a Tg of greater than or equal to 40°C,
- b) a block with a Tg of less than or equal to 20°C,
- c) a block with a Tg of between 20 and 40°C, and the second block being chosen from a category a), b) or c) different from the first block;

and the said block polymer having a polydispersity index I of greater than 2.8,
the said polymer being such that, when it is in sufficient amount in the composition, the mean gloss at 20° of a deposit of the said composition, once spread onto a support, is greater than or equal to 30 out of 100.

**2.** Liquid cosmetic composition containing a cosmetically acceptable organic liquid medium and a film-forming linear block ethylenic polymer free of styrene units,
the said block polymer containing first and second blocks linked together via an intermediate segment which is a random polymer comprising at least one constituent monomer of the first block and at least one constituent monomer of the second block,
the first block of the polymer being chosen from:

- a) a block with a Tg of greater than or equal to 40°C,
- b) a block with a Tg of less than or equal to 20°C,
- c) a block with a Tg of between 20 and 40°C, and the second block being chosen from a category a), b) or c) different from the first block;

and the said block polymer having a polydispersity index I of greater than 2.8,
the polymer being such that, when it is in sufficient amount in the composition, the mean gloss at 20° of a deposit of the said composition, once spread onto a support, is greater than or equal to 30 out of 100.

**3.** Cosmetic composition according to Claim 1 or 2, **characterized in that** the block polymer is an ethylenic polymer derived from aliphatic ethylenic monomers comprising a carbon-carbon double bond and at least one ester -COO- or amide -CON- group.

**4.** Cosmetic composition according to one of the preceding claims, **characterized in that** the polymer is not soluble at an active material content of at least 1% by weight in water or in a mixture of water and of linear or branched lower monoalcohols containing from 2 to 5 carbon atoms, without pH modification, at room temperature (25°C).

**5.** Composition according to the preceding claim, **characterized in that** the first and second blocks are linked together via an intermediate segment with a glass transition temperature that is between the glass transition temperatures of the first and second blocks.

**6.** Cosmetic composition according to any one of the preceding claims, **characterized in that** the block polymer contains first and second blocks that are incompatible in the said organic liquid medium.

**7.** Composition according to any one of the preceding claims, **characterized in that** the block with a Tg of greater than or equal to 40°C is totally or partially derived from one or more monomers, which are such that the homopolymer prepared from these monomers has a glass transition temperature of greater than or equal to 40°C.

**8.** Composition according to the preceding claim, **characterized in that** the monomers whose corresponding homopolymer has a glass transition temperature of greater than or equal to 40°C are chosen from the following monomers:

- methacrylates of formula $CH_2 = C(C_H3)-COOR_1$ in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2 = CH-COOR_2$ in which $R_2$ represents a $C_4$ to $C_{12}$ cycloalkyl group such as isobornyl acrylate or a tert-butyl group,
- (meth)acrylamides of formula:

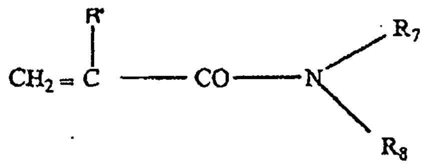

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl,
- and mixtures thereof.

9. Composition according to Claim 7 or 8, **characterized in that** the monomers whose corresponding homopolymer has a glass transition temperature of greater than or equal to 40°C are chosen from methyl methacrylate, isobutyl (meth)acrylate and isobornyl (meth)acrylate, and mixtures thereof.

10. Composition according to any one of Claims 1 to 6, **characterized in that** the block with a Tg of less than or equal to 20°C is derived totally or partially from one or more monomers, which are such that the homopolymer prepared from these monomers has a glass transition temperature of less than or equal to 20°C.

11. Composition according to the preceding claim, **characterized in that** the monomers whose corresponding homopolymer has a glass transition temperature of less than or equal to 20°C are chosen from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more hetero atoms chosen from O, N and S is (are) optionally intercalated;
- methacrylates of formula $CH_2 = C(CH_3)-COOR_4$,
$R_4$ representing a linear or branched $C_6$ to $C_{12}$ unsubstituted alkyl group, in which one or more hetero atoms chosen from O, N and S is (are) optionally intercalated;
- vinyl esters of formula $R_5-CO-O-CH = CH_2$ in which $R_5$ represents a linear or branched $C_4$ to $C_{12}$ alkyl group;
- $C_4$ to $C_{12}$ alkyl vinyl ethers,
- N- ($C_4$ to $C_{12}$) alkyl acrylamides, such as N-octylacrylamide,
- and mixtures thereof.

12. Composition according to Claim 10 or 11, **characterized in that** the monomers whose corresponding homopolymer has a glass transition temperature of less than or equal to 20°C are chosen from alkyl acrylates whose alkyl chain contains from 1 to 10 carbon atoms, with the exception of the tert-butyl group.

13. Composition according to any one of Claims 1 to 6, **characterized in that** the block with a Tg of between 20 and 40°C is totally or partially derived from one or more monomers, which are such that the homopolymer prepared from these monomers has a glass transition temperature of between 20 and 40°C.

14. Composition according to the preceding claim, **characterized in that** the block with a Tg of between 20 and 40°C is totally or partially derived from monomers which are such that the corresponding homopolymer has a Tg of greater than or equal to 40°C and from monomers which are such that the corresponding homopolymer has a Tg of less than or equal to 20°C.

15. Composition according to Claim 13 or 14, **characterized in that** the block with a Tg of between 20 and 40°C is

totally or partially derived from monomers chosen from methyl methacrylate, isobornyl acrylate and methacrylate, butyl acrylate and 2-ethylhexyl acrylate, and mixtures thereof.

**16.** Composition according to any one of Claims 1 to 12, **characterized in that** it comprises a block polymer comprising at least one first block and at least one second block, the first block having a glass transition temperature (Tg) of greater than or equal to 40°C and the second block having a glass transition temperature of less than or equal to 20°C.

**17.** Composition according to the preceding claim, **characterized in that** the first block is totally or partially derived from one or more monomers which are such that the homopolymer prepared from these monomers has a glass transition temperature of greater than or equal to 40°C.

**18.** Composition according to the preceding claim, **characterized in that** the first block is a copolymer derived from monomers which are such that the homopolymer prepared from these monomers has a glass transition temperature of greater than or equal to 40°C.

**19.** Composition according to Claim 17 or 18, **characterized in that** the monomers whose corresponding homopolymer has a glass transition temperature of greater than or equal to 40°C are chosen from the following monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2 = CH\text{-}COOR_2$ in which $R_2$ represents a $C_4$ to $C_{12}$ cycloalkyl group such as isobornyl acrylate or a tert-butyl group,
- (meth)acrylamides of formula:

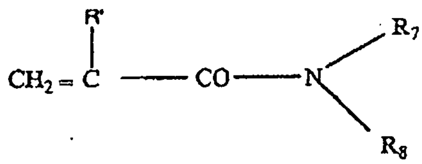

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl,
- and mixtures thereof.

**20.** Composition according to one of Claims 17 to 19, **characterized in that** the monomers whose corresponding homopolymer has a glass transition temperature of greater than or equal to 40°C are chosen from methyl methacrylate, isobutyl methacrylate and isobornyl (meth)acrylate, and mixtures thereof.

**21.** Composition according to one of Claims 16 to 19, **characterized in that** the proportion of the first block ranges from 20% to 90%, better still from 30% to 80% and even better from 50% to 70% by weight of the polymer.

**22.** Composition according to one of Claims 16 to 21, **characterized in that** the second block is totally or partially derived from one or more monomers which are such that the homopolymer prepared from these monomers has a glass transition temperature of less than or equal to 20°C.

**23.** Composition according to one of Claims 16 to 22, **characterized in that** the second block is a homopolymer derived from monomers which are such that the homopolymer prepared from these monomers has a glass transition temperature of less than or equal to 20°C.

**24.** Composition according to Claim 22 or 23, **characterized in that** the monomers whose corresponding homopolymer has a glass transition temperature of less than or equal to 20°C are chosen from the following monomers:

- acrylates of formula $CH_2 = CHCOOR_3$,
$R_3$ representing a linear or branched $C_1$ to $C_{12}$ unsubstituted alkyl group, with the exception of the tert-butyl

group, in which one or more hetero atoms chosen from O, N and S is (are) optionally intercalated;

- methacrylates of formula $CH_2 = C(CH_3)-COOR_4$,

$R_4$ representing a linear or branched $C_6$ to $C_{12}$ unsubstituted alkyl group, in which one or more hetero atoms chosen from O, N and S is (are) optionally intercalated;

- vinyl esters of formula $R_5-CO-O-CH = CH_2$ in which $R_5$ represents a linear or branched $C_4$ to $C_{12}$ alkyl group;
- $C_4$ to $C_{12}$ alkyl vinyl ethers,
- N- ($C_4$ to $C_{12}$) alkyl acrylamides, such as N-octylacrylamide,
- and mixtures thereof.

**25.** Composition according to one of Claims 22 to 24, **characterized in that** the monomers whose corresponding homopolymer has a glass transition temperature of less than or equal to 20°C are chosen from alkyl acrylates whose alkyl chain contains from 1 to 10 carbon atoms, with the exception of the tert-butyl group.

**26.** Composition according to one of Claims 16 to 25, **characterized in that** the proportion of the second block with a Tg of less than or equal to 20°C ranges from 5% to 75%, better still from 15% to 50% and even better from 25% to 45% by weight of the polymer.

**27.** Composition according to one of Claims 1 to 12, **characterized in that** it comprises a block polymer comprising at least one first block and at least one second block, the first block having a glass transition temperature (Tg) of between 20 and 40°C and the second block having a glass transition temperature of less than or equal to 20°C or a glass transition temperature of greater than or equal to 40°C.

**28.** Composition according to the preceding claim, **characterized in that** the first block with a Tg of between 20 and 40°C is totally or partially derived from one or more monomers which are such that the homopolymer prepared from these monomers has a glass transition temperature of between 20 and 40°C.

**29.** Composition according to Claim 27 or 28, **characterized in that** the first block with a Tg of between 20 and 40°C is a copolymer derived from monomers which are such that the corresponding homopolymer has a Tg of greater than or equal to 40°C and from monomers which are such that the corresponding homopolymer has a Tg of less than or equal to 20°C.

**30.** Composition according to one of Claims 27 to 29, **characterized in that** the first block with a Tg of between 20 and 40°C is derived from monomers chosen from methyl methacrylate, isobornyl acrylate and methacrylate, butyl acrylate and 2-ethylhexyl acrylate, and mixtures thereof.

**31.** Composition according to one of Claims 27 to 30, **characterized in that** the proportion of the first block with a Tg of between 20 and 40°C ranges from 10% to 85%, better still from 30% to 80% and even better from 50% to 70% by weight of the polymer.

**32.** Composition according to any one of Claims 27 to 31, **characterized in that** the second block has a Tg of greater than or equal to 40°C and is totally or partially derived from one or more monomers which are such that the homopolymer prepared from these monomers has a glass transition temperature of greater than or equal to 40°C.

**33.** Composition according to any one of Claims 27 to 32, **characterized in that** the second block has a Tg of greater than or equal to 40°C and is a homopolymer derived from monomers which are such that the homopolymer prepared from these monomers has a glass transition temperature of greater than or equal to 40°C.

**34.** Composition according to either of Claims 32 and 33, **characterized in that** the monomers whose corresponding homopolymer has a glass transition temperature of greater than or equal to 40°C are chosen from the following monomers:

- methacrylates of formula $CH_2 = C(CH_3)-COOR_1$ in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2 = CH-COOR_2$ in which $R_2$ represents a $C_4$ to $C_{12}$ cycloalkyl group such as isobornyl

acrylate or a tert-butyl group,
- (meth)acrylamides of formula:

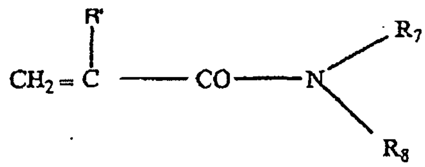

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl,

- and mixtures thereof.

**35.** Composition according to one of Claims 32 to 35, **characterized in that** the monomers whose corresponding homopolymer has a glass transition temperature of greater than or equal to 40°C are chosen from methyl methacrylate, isobutyl methacrylate and isobornyl (meth)acrylate, and mixtures thereof.

**36.** Composition according to one of Claims 32 to 35, **characterized in that** the proportion of the second block with a Tg of greater than or equal to 40°C ranges from 10% to 85%, preferably from 20% to 70% and better still from 30% to 70% by weight of the polymer.

**37.** Composition according to one of Claims 27 to 31, **characterized in that** the second block has a Tg of less than or equal to 20°C and is totally or partially derived from one or more monomers which are such that the homopolymer prepared from these monomers has a glass transition temperature of less than or equal to 20°C.

**38.** Composition according to one of Claims 27 to 31, **characterized in that** the second block has a Tg of less than or equal to 20°C and is a homopolymer derived from monomers which are such that the homopolymer prepared from these monomers has a glass transition temperature of less than or equal to 20°C.

**39.** Composition according to Claim 37 or 38, **characterized in that** the monomers whose corresponding homopolymer has a glass transition temperature of less than or equal to 20°C are chosen from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more hetero atoms chosen from O, N and S is (are) optionally intercalated;
- methacrylates of formula $CH_2 = C(CH_3)\text{-}COOR_4$,
$R_4$ representing a linear or branched $C_6$ to $C_{12}$ unsubstituted alkyl group, in which one or more hetero atoms chosen from O, N and S is (are) optionally intercalated;
- vinyl esters of formula $R_5\text{-CO-O-CH} = CH_2$ in which $R_5$ represents a linear or branched $C_4$ to $C_{12}$ alkyl group;
- $C_4$ to $C_{12}$ alkyl vinyl ethers,
- N- ($C_4$ to $C_{12}$) alkyl acrylamides, such as N-octylacrylamide,
- and mixtures thereof.

**40.** Composition according to one of Claims 37 to 39, **characterized in that** the monomers whose homopolymers have glass transition temperatures of less than or equal to 20°C are chosen from alkyl acrylates whose alkyl chain contains from 1 to 10 carbon atoms, with the exception of the tert-butyl group.

**41.** Composition according to one of Claims 37 to 40, **characterized in that** the proportion of the block with a glass transition temperature of less than or equal to 20°C ranges from 10% to 85%, better still from 20% to 70% and even better from 20% to 50% by weight of the polymer.

**42.** Cosmetic composition according to any one of the preceding claims, **characterized in that** the first block and/or the second block comprises at least one additional monomer.

**43.** Composition according to the preceding claim, **characterized in that** the additional monomer is chosen from hydrophilic monomers and ethylenically unsaturated monomers comprising one or more silicon atoms, and mixtures thereof.

**44.** Composition according to Claim 42 or 43, **characterized in that** the additional monomer is chosen from:

a) hydrophilic monomers such as:

- ethylenically unsaturated monomers comprising at least one carboxylic or sulfonic acid function, for instance:

acrylic acid, methacrylic acid, crotonic acid, maleic anhydride, itaconic acid, fumaric acid, maleic acid, acrylamidopropanesulfonic acid, vinylbenzoic acid, vinylphosphoric acid, and salts thereof,

- ethylenically unsaturated monomers comprising at least one tertiary amine function, for instance 2-vinylpyridine, 4-vinylpyridine, dimethylaminoethyl methacrylate, diethylaminoethyl methacrylate and dimethylaminopropylmethacrylamide, and salts thereof,
- methacrylates of formula $CH_2 = C(CH_3)\text{-}COOR_6$ in which $R_6$ represents a linear or branched alkyl group containing from 1 to 4 carbon atoms, such as a methyl, ethyl, propyl or isobutyl group, the said alkyl group being substituted with one or more substituents chosen from hydroxyl groups (for instance 2-hydroxypropyl methacrylate and 2-hydroxyethyl methacrylate) and halogen atoms (Cl, Br, I or F), such as trifluoroethyl methacrylate,
- methacrylates of formula $CH_2 = C(CH_3)\text{-}COOR_9$,
$R_9$ representing a linear or branched $C_6$ to $C_{12}$ alkyl group in which one or more hetero atoms chosen from O, N and S is (are) optionally intercalated, the said alkyl group being substituted with one or more substituents chosen from hydroxyl groups and halogen atoms (Cl, Br, I or F);
- acrylates of formula $CH_2 = CHCOOR_{10}$,
$R_{10}$ representing a linear or branched $C_1$ to $C_{12}$ alkyl group substituted with one or more substituents chosen from hydroxyl groups and halogen atoms (Cl, Br, I or F), such as 2-hydroxypropyl acrylate and 2-hydroxyethyl acrylate, or $R_{10}$ represents a ($C_1$-$C_{12}$) alkyl-O-POE (polyoxyethylene) with repetition of the oxyethylene unit 5 to 30 times, for example methoxy-POE, or $R_{10}$ represents a polyoxyethylenated group comprising from 5 to 30 ethylene oxide units, and

b) ethylenically unsaturated monomers comprising one or more silicon atoms, such as methacryloxypropyltrimethoxysilane and methacryloxypropyltris(trimethylsiloxy)silane,

- and mixtures thereof.

**45.** Composition according to either of Claims 42 and 43, **characterized in that** each of the first and second blocks comprises at least one additional monomer chosen from acrylic acid, (meth)acrylic acid and trifluoroethyl methacrylate, and mixtures thereof.

**46.** Composition according to either of Claims 42 and 43, **characterized in that** each of the first and second blocks comprises at least one monomer chosen from (meth)acrylic acid esters and optionally at least one additional monomer such as (meth)acrylic acid, and mixtures thereof.

**47.** Composition according to either of Claims 42 and 43, **characterized in that** each of the first and second blocks is totally derived from at least one monomer chosen from (meth)acrylic acid esters and optionally from at least one additional monomer such as (meth)acrylic acid, and mixtures thereof.

**48.** Composition according to one of Claims 42 to 47, **characterized in that** the additional monomer(s) represent(s) from 1% to 30% by weight relative to the total weight of the first and/or second blocks.

**49.** Composition according to any one of the preceding claims, **characterized in that** the difference between the glass transition temperatures (Tg) of the first and second blocks is greater than 10°C, better still greater than 20°C, preferably greater than 30°C and better still greater than 40°C.

**50.** Composition according to any one of the preceding claims, **characterized in that** the block polymer has a polydispersity index of between 2.8 and 6.

**51.** Composition according to one of the preceding claims, **characterized in that** the block polymer has a weight-average mass (Mw) of less than or equal to 300 000.

**52.** Composition according to the preceding claim, **characterized in that** the weight-average mass (Mw) ranges from 35 000 to 200 000 and better still from 45 000 to 150 000.

**53.** Composition according to the preceding claim, **characterized in that** the number-average mass (Mn) is less than or equal to 70 000.

**54.** Composition according to one of Claims 51 to 53, the number-average mass (Mn) of which ranges from 10 000 to 60 000 and better still from 12 000 to 50 000.

**55.** Composition according to one of the preceding claims, **characterized in that** the mean gloss of the composition measured at 20° is greater than or equal to 30, even better greater than or equal to 35, better still greater than or equal to 40, better still greater than or equal to 45, better still greater than or equal to 50 out of 100, better still greater than or equal to 55, better still greater than or equal to 60, better still greater than or equal to 65, better still greater than or equal to 70, or even better still greater than or equal to 75 out of 100.

**56.** Composition according to one of the preceding claims, **characterized in that** the mean gloss of the composition, once spread onto a support, measured at 60°, is greater than or equal to 50, better still greater than or equal to 60, better still greater than or equal to 65, better still greater than or equal to 70, better still greater than or equal to 75, better still greater than or equal to 80, better still greater than or equal to 85 or even better still greater than or equal to 90 out of 100.

**57.** Composition according to one of the preceding claims, **characterized in that** the mean gloss of the composition measured at 20° is greater than or equal to 35, preferably 40, 45 or 50 out of 100, and/or the gloss of the composition measured at 60° is greater than or equal to 65, 70 or 75 out of 100.

**58.** Composition according to one of the preceding claims, **characterized in that** the gloss of the composition measured at 20° is greater than or equal to 60, preferably 65, 70 or 75 out of 100, and/or the gloss of the composition measured at 60° is greater than or equal to 80, 85 or 90 out of 100.

**59.** Composition according to one of the preceding claims, **characterized in that** it comprises from 0.1% to 60% by weight of active material, preferably from 5% to 50% by weight and more preferably from 10% to 40% by weight, of polymer.

**60.** Cosmetic composition according to any one of the preceding claims, **characterized in that** it also comprises one or more dyestuffs chosen from watersoluble dyes and pulverulent dyestuffs, such as pigments, nacres and flakes.

**61.** Cosmetic composition according to any one of the preceding claims, **characterized in that** it is in the form of a suspension, a dispersion, a solution, a gel, an emulsion, especially an oil-in-water (O/W) or water-in-oil (W/O) emulsion, or a multiple emulsion (W/O/W or polyol/O/W or O/W/O), or in the form of a cream, a mousse, a dispersion of vesicles, especially of ionic or nonionic lipids, a two-phase or multi-phase lotion, or a paste, especially a soft paste or an anhydrous paste.

**62.** Cosmetic composition according to any one of the preceding claims, **characterized in that** it is in anhydrous form.

**63.** Cosmetic composition according to any one of the preceding claims, **characterized in that** it is a makeup or care composition for keratin materials.

**64.** Cosmetic composition according to one of the preceding claims, **characterized in that** it is a lip makeup product.

**65.** Cosmetic composition according to one of the preceding claims, **characterized in that** it is an eye makeup product.

**66.** Cosmetic composition according to one of the preceding claims, **characterized in that** it is a nail makeup product.

**67.** Cosmetic assembly comprising:

a) a container delimiting at least one compartment, the said container being closed by a closing member; and
b) a composition placed inside the said compartment, the composition being in accordance with any one of the preceding claims.

**68.** Cosmetic assembly according to the preceding claim, **characterized in that** the container is at least partially formed from at least one thermoplastic material.

**69.** Cosmetic assembly according to Claim 67, **characterized in that** the container is at least partially formed from at least one non-thermoplastic material, especially from glass or metal.

**70.** Assembly according to any one of Claims 67 to 69, **characterized in that**, in the closed position of the container, the closing member is screwed onto the container.

**71.** Assembly according to any one of Claims 67 to 69, **characterized in that**, in the closed position of the container, the closing member is coupled to the container other than by screwing, especially by click-fastening, bonding or welding.

**72.** Assembly according to any one of Claims 67 to 71, **characterized in that** the composition is substantially at atmospheric pressure inside the compartment.

**73.** Assembly according to any one of Claims 67 to 71, **characterized in that** the composition is pressurized inside the container.

**74.** Cosmetic process for making up or caring for keratin materials, comprising the application to the keratin materials of a cosmetic composition according to one of Claims 1 to 66.

**Patentansprüche**

**1.** Flüssige kosmetische Zusammensetzung, die ein kosmetisch akzeptables, flüssiges organisches Medium und ein filmbildendes, lineares, ethylenisches, nicht elastomeres Sequenzpolymer enthält,
wobei das Sequenzpolymer eine erste Sequenz und eine zweite Sequenz enthält, die über ein Zwischensegment aneinander gebunden sind, bei dem es sich um ein statistisches Polymer handelt, das mindestens ein die erste Sequenz aufbauendes Monomer und mindestens ein die zweite Sequenz aufbauendes Monomer umfasst,
wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40 °C;

und die zweite Sequenz aus einer von der ersten Sequenz verschiedenen Gruppe a), b) oder c) ausgewählt ist;
wobei das Sequenzpolymer einen Polydispersitätsindex I über 2,8 aufweist,
und wobei das Polymer so ist, dass der mittlere Glanz einer Abscheidung der Zusammensetzung bei 20°, wenn es in der Zusammensetzung in einer ausreichenden Menge enthalten ist, nach dem Auftragen auf einem Träger größer oder gleich 30 von 100 ist.

**2.** Flüssige kosmetische Zusammensetzung, die ein kosmetisch akzeptables, flüssiges organisches Medium und ein filmbildendes, lineares, ethylenisches Sequenzpolymer frei von Styrol enthält,
wobei das Sequenzpolymer eine erste Sequenz und eine zweite Sequenz enthält, die über ein Zwischensegment aneinander gebunden sind, bei dem es sich um ein statistisches Polymer handelt, das mindestens ein die erste Sequenz aufbauendes Monomer und mindestens ein die zweite Sequenz aufbauendes Monomer umfasst,
wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40 °C;

und die zweite Sequenz aus einer von der ersten Sequenz verschiedenen Gruppe a), b) oder c) ausgewählt ist; wobei das Sequenzpolymer einen Polydispersitätsindex I über 2,8 aufweist, und wobei das Polymer so ist, dass der mittlere Glanz einer Abscheidung der Zusammensetzung bei 20°, wenn es in der Zusammensetzung in einer ausreichenden Menge enthalten ist, nach dem Auftragen auf einem Träger größer oder gleich 30 von 100 ist.

3. Kosmetische Zusammensetzung nach Anspruch 1 oder 2, **dadurch gekennzeichnet, dass** das Sequenzpolymer ein ethylenisches Polymer ist, das von aliphatischen ethylenischen Monomeren abgeleitet ist, die eine Kohlenstoff-Kohlenstoff-Doppelbindung und mindestens eine Estergruppe -COO- oder Amidgruppe -CON- aufweisen.

4. Kosmetische Zusammensetzung nach einem der vorhergehenden Ansprüche, **dadurch gekennzeichnet, dass** das Polymer bei einem Gehalt der wirksamen Substanz von mindestens 1 Gew.-% in Wasser oder einem Gemisch von Wasser und linearen oder verzweigten, niederen Monoalkoholen mit 2 bis 5 Kohlenstoffatomen ohne pH-Wert-Änderung bei Raumtemperatur (25 °C) nicht löslich ist.

5. Zusammensetzung nach dem vorhergehenden Anspruch, **dadurch gekennzeichnet, dass** die erste Sequenz und die zweite Sequenz über ein Zwischensegment aneinander gebunden sind, das eine Glasübergangstemperatur aufweist, die zwischen den Glasübergangstemperaturen der ersten und der zweiten Sequenz liegt.

6. Kosmetische Zusammensetzung nach einem der vorhergehenden Ansprüche, **dadurch gekennzeichnet, dass** das Sequenzpolymer eine erste Sequenz und eine zweite Sequenz aufweist, die in dem flüssigen organischen Medium nicht miteinander kompatibel sind.

7. Kosmetische Zusammensetzung nach einem der vorhergehenden Ansprüche, **dadurch gekennzeichnet, dass** die Sequenz mit einer Tg von größer oder gleich 40 °C ganz oder teilweise von einem oder mehreren Monomeren stammt, die so sind, dass das aus diesen Monomeren hergestellte Homopolymer eine Glasübergangstemperatur von größer oder gleich 40 °C aufweist.

8. Zusammensetzung nach dem vorhergehenden Anspruch, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

· Methacrylaten der Formel $CH_2=C(CH_3)-COOR_1$,
worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
· Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
· (Meth)acrylamiden der Formel:

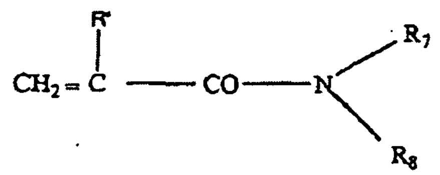

worin $R_7$ und $R_8$, die gleich oder verschieden sind, jeweils ein Wasserstoffatom oder eine lineare oder verzweigte $C_{1-12}$-Alkylgruppe, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
· und deren Gemischen.

9. Zusammensetzung nach Anspruch 7 oder 8, **dadurch gekennzeichnet, dass** die Monomere, deren entsprechendes Homopolymer eine Glasübergangstemperatur von größer oder gleich 40 °C aufweist, unter Methylmethacrylat, Isobutyl(meth)acrylat und Isobornyl(meth)acrylat und deren Gemischen ausgewählt sind.

10. Zusammensetzung nach einem der Ansprüche 1 bis 6, **dadurch gekennzeichnet, dass** die Sequenz mit einer Tg von kleiner oder gleich 20 °C ganz oder teilweise von einem oder mehreren Monomeren stammt, die so sind, dass

das aus diesen Monomeren hergestellte Homopolymer eine Glasübergangstemperatur von kleiner oder gleich 20 °C aufweist.

**11.** Zusammensetzung nach dem vorhergehenden Anspruch, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

· Acrylaten der Formel $CH_2$=CHCOOR$_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
· Methacrylaten der Formel $CH_2$=C($CH_3$)-COOR$_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
· Vinylestern der Formel $R_5$-CO-O-CH=$CH_2$,
worin $R_5$ eine lineare oder verzweigte Alkylgruppe mit 4 bis 12 Kohlenstoffatomen bedeutet,
· Alkyl($C_{4-12}$)vinylethern,
· N-Alkyl($C_{4-12}$)acrylamiden, wie N-Octylacrylamid,
· und deren Gemischen.

**12.** Zusammensetzung nach Anspruch 10 oder 11, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, mit Ausnahme der tert-Butylgruppe.

**13.** Zusammensetzung nach einem der Ansprüche 1 bis 6, **dadurch gekennzeichnet, dass** die Sequenz mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweist.

**14.** Zusammensetzung nach dem vorhergehenden Anspruch,
**dadurch gekennzeichnet, dass** die Sequenz mit einer Tg zwischen 20 und 40°C ganz oder teilweise von Monomeren, die so sind, dass das entsprechende Homopolymer eine Tg von größer oder gleich 40 °C aufweist, und von Monomeren abgeleitet ist, die so sind, dass das entsprechende Homopolymer eine Tg von kleiner oder gleich 20 °C aufweist.

**15.** Zusammensetzung nach Anspruch 13 oder 14, **dadurch gekennzeichnet, dass** die Sequenz mit einer Tg zwischen 20 und 40°C ganz oder teilweise von Monomeren abgeleitet ist, die unter Methylmethacrylat, Isobornylacrylat, Isobornylmethacrylat, Butylacrylat und 2-Ethylhexylacrylat und deren Gemischen ausgewählt sind.

**16.** Zusammensetzung nach einem der Ansprüche 1 bis 12, **dadurch gekennzeichnet, dass** das Sequenzpolymer zumindest eine erste Sequenz und zumindest eine zweite Sequenz umfasst, wobei die erste Sequenz eine Glasübergangstemperatur (Tg) von größer oder gleich 40 °C und die zweiten Sequenz eine Glasübergangstemperatur von kleiner oder gleich 20 °C aufweist.

**17.** Zusammensetzung nach dem vorhergehenden Anspruch, **dadurch gekennzeichnet, dass** die erste Sequenz ganz oder teilweise von einem oder mehreren Monomeren stammt, die so sind, dass das aus diesen Monomeren hergestellte Homopolymer eine Glasübergangstemperatur von größer oder gleich 40 °C aufweist.

**18.** Zusammensetzung nach dem vorhergehenden Anspruch, **dadurch gekennzeichnet, dass** die erste Sequenz ein Copolymer ist, das von Monomeren abgeleitet ist, die so sind, dass das aus diesen Monomeren hergestellte Homopolymer eine Glasübergangstemperatur von größer oder gleich 40 °C aufweist.

**19.** Zusammensetzung nach Anspruch 17 oder 18, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

· Methacrylaten der Formel $CH_2$=C($CH_3$)-COOR$_1$,
worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,

· Acrylaten der Formel $CH_2$=CH-$COOR_2$,
worin $R_2$ eine Cycloalkylgruppe mit 4 bis 12 Kohlenstoffatomen, wie Isobornylacrylat, oder tert-Butyl bedeutet,
· (Meth)acrylamiden der Formel:

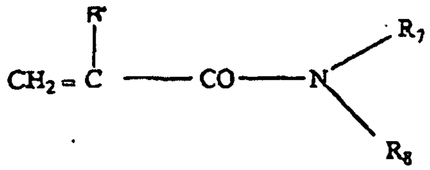

worin $R_7$ und $R_8$, die gleich oder verschieden sind, jeweils ein Wasserstoffatom oder eine lineare oder verzweigte $C_{1-12}$-Alkylgruppe, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
· und deren Gemischen.

**20.** Zusammensetzung nach einem der Ansprüche 17 bis 19, **dadurch gekennzeichnet, dass** die Monomere, deren entsprechendes Homopolymer eine Glasübergangstemperatur von größer oder gleich 40 °C aufweist, unter Methylmethacrylat, Isobutylmethacrylat und Isobornyl(meth)acrylat und deren Gemischen ausgewählt sind.

**21.** Zusammensetzung nach einem der Ansprüche 16 bis 19, **dadurch gekennzeichnet, dass** der Anteil der ersten Sequenz im Bereich von 20 bis 90 Gew.-% des Polymers, besser im Bereich von 30 bis 80 Gew.-% und noch besser im Bereich von 50 bis 70 Gew.-% liegt.

**22.** Zusammensetzung nach einem der Ansprüche 16 bis 21, **dadurch gekennzeichnet, dass** die zweite Sequenz ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**23.** Zusammensetzung nach einem der Ansprüche 16 bis 22, **dadurch gekennzeichnet, dass** die zweite Sequenz ein Homopolymer ist, das von Monomeren abgeleitet ist, die so sind, dass das aus diesen Monomeren hergestellte Homopolymer eine Glasübergangstemperatur von kleiner oder gleich 20 °C aufweist.

**24.** Zusammensetzung nach Anspruch 22 oder 23, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

· Acrylaten der Formel $CH_2$=$CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
· Methacrylaten der Formel $CH_2$=C($CH_3$)-$COOR_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
· Vinylestern der Formel $R_5$-CO-O-CH=$CH_2$,
worin $R_5$ eine lineare oder verzweigte Alkylgruppe mit 4 bis 12 Kohlenstoffatomen bedeutet,
· Alkyl($C_{4-12}$)vinylethern,
· N-Alkyl($C_{4-12}$)acrylamiden, wie N-Octylacrylamid,
· und deren Gemischen.

**25.** Zusammensetzung nach einem der Ansprüche 22 bis 24, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, wobei die tert-Butylgruppe ausgenommen ist.

**26.** Zusammensetzung nach einem der Ansprüche 16 bis 25, **dadurch gekennzeichnet, dass** der Anteil der zweiten Sequenz mit einer Tg von kleiner oder gleich 20 °C im Bereich von 5 bis 75 Gew.-% des Polymers, besser im Bereich von 15 bis 50 Gew.-% und noch besser im Bereich von 25 bis 45 Gew.-% liegt.

**27.** Zusammensetzung nach einem der Ansprüche 1 bis 12, **dadurch gekennzeichnet, dass** sie ein Sequenzpolymer enthält, das zumindest eine erste Sequenz und zumindest eine zweite Sequenz umfasst, wobei die erste Sequenz eine Glasübergangstemperatur (Tg) zwischen 20 °C und 40 °C aufweist und die zweite Sequenz eine Glasübergangstemperatur von kleiner oder gleich 20 °C oder einer Glasübergangstemperatur von größer oder gleich 40 °C aufweist.

**28.** Zusammensetzung nach dem vorhergehenden Anspruch, **dadurch gekennzeichnet, dass** die Sequenz mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweist.

**29.** Zusammensetzung nach Anspruch 27 oder 28, **dadurch gekennzeichnet, dass** die erste Sequenz mit einer Tg zwischen 20 und 40°C ein Copolymer ist, das von Monomeren, die so sind, dass das entsprechende Homopolymer eine Tg von größer oder gleich 40 °C aufweist, und Monomeren abgeleitet ist, die so sind, dass das entsprechende Homopolymer eine Tg von kleiner oder gleich 20 °C aufweist.

**30.** Zusammensetzung nach einem der Ansprüche 27 bis 29, **dadurch gekennzeichnet, dass** die erste Sequenz mit einer Tg zwischen 20 und 40°C von Monomeren abgeleitet ist, die unter Methylmethacrylat, Isobornylacrylat, Isobornylmethacrylat, Butylacrylat und 2-Ethylhexylacrylat und deren Gemischen ausgewählt sind.

**31.** Zusammensetzung nach einem der Ansprüche 27 bis 30, **dadurch gekennzeichnet, dass** der Anteil der ersten Sequenz mit einer Tg zwischen 20 und 40 °C im Bereich von 10 bis 85 Gew.-% des Polymers, besser im Bereich von 30 bis 80 Gew.-% und noch besser im Bereich von 50 bis 70 Gew.-% liegt.

**32.** Zusammensetzung nach einem der Ansprüche 27 bis 31, **dadurch gekennzeichnet, dass** die zweite Sequenz mit einer Tg von größer oder gleich 40 °C ganz oder teilweise von einem oder mehreren Monomeren stammt, die so sind, dass das aus diesen Monomeren hergestellte Homopolymer eine Glasübergangstemperatur von größer oder gleich 40 °C aufweist.

**33.** Zusammensetzung nach einem der Ansprüche 27 bis 32, **dadurch gekennzeichnet, dass** die zweite Sequenz eine Tg von größer oder gleich 40 °C aufweist und ein Homopolymer ist, das von Monomeren abgeleitet ist, die so sind, dass das aus diesen Monomeren hergestellte Homopolymer eine Glasübergangstemperatur von größer oder gleich 40 °C aufweist.

**34.** Zusammensetzung nach einem der Ansprüche 32 oder 33, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

· Methacrylaten der Formel $CH_2=C(CH_3)-COOR_1$,
worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
· Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
· (Meth)acrylamiden der Formel:

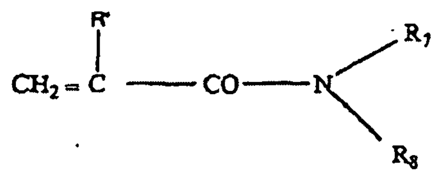

worin $R_7$ und $R_8$, die gleich oder verschieden sind, jeweils ein Wasserstoffatom oder eine lineare oder verzweigte $C_{1-12}$-Alkylgruppe, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
· und deren Gemischen.

**35.** Zusammensetzung nach einem der Ansprüche 32 bis 35, **dadurch gekennzeichnet, dass** die Monomere, deren

entsprechendes Homopolymer eine Glasübergangstemperatur von größer oder gleich 40 °C aufweist, unter Methylmethacrylat, Isobutylmethacrylat und Isobornyl(meth)acrylat und deren Gemischen ausgewählt sind.

**36.** Zusammensetzung nach einem der Ansprüche 32 bis 35, **dadurch gekennzeichnet, dass** der Anteil der zweiten Sequenz mit einer Tg von größer oder gleich 40 °C im Bereich von 10 bis 85 Gew.-%, vorzugsweise 20 bis 70 Gew.-% und besser 30 bis 70 Gew.-% des Polymers liegt.

**37.** Zusammensetzung nach einem der Ansprüche 27 bis 31, **dadurch gekennzeichnet, dass** die zweite Sequenz eine Tg von kleiner oder gleich 20 °C aufweist und ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**38.** Zusammensetzung nach einem der Ansprüche 27 bis 31, **dadurch gekennzeichnet, dass** die zweite Sequenz eine Tg von kleiner oder gleich 20 °C aufweist und ein Homopolymer ist, das von Monomeren abgeleitet ist, die so sind, dass das aus diesen Monomeren hergestellte Homopolymer eine Glasübergangstemperatur von kleiner oder gleich 20 °C aufweist.

**39.** Zusammensetzung nach Anspruch 37 oder 38, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

· Acrylaten der Formel $CH_2=CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
· Methacrylaten der Formel $CH_2=C(CH_3)-COOR_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
· Vinylestern der Formel $R_5-CO-O-CH=CH_2$,
worin $R_5$ eine lineare oder verzweigte Alkylgruppe mit 4 bis 12 Kohlenstoffatomen bedeutet,
· Alkyl($C_{4-12}$)vinylethern,
· N-Alkyl($C_{4-12}$)acrylamiden, wie N-Octylacrylamid,
· und deren Gemischen.

**40.** Zusammensetzung nach einem der Ansprüche 37 bis 39, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, mit Ausnahme der tert-Butylgruppe.

**41.** Zusammensetzung nach einem der Ansprüche 37 bis 40, **dadurch gekennzeichnet, dass** der Anteil der Sequenz mit einer Glasübergangstemperatur von kleiner oder gleich 20 °C im Bereich von 10 bis 85 Gew.-% des Polymers, besser im Bereich von 20 bis 70 Gew.-% und noch besser im Bereich von 20 bis 50 Gew.-% liegt.

**42.** Kosmetische Zusammensetzung nach einem der vorhergehenden Ansprüche, **dadurch gekennzeichnet, dass** die erste Sequenz und/oder die zweite Sequenz mindestens ein ergänzendes Monomer aufweisen.

**43.** Zusammensetzung nach dem vorhergehenden Anspruch, **dadurch gekennzeichnet, dass** das ergänzende Monomer unter den hydrophilen Monomeren, Monomeren mit ethylenisch ungesättigter Bindung, die ein oder mehrere Siliciumatome enthalten, und deren Gemischen ausgewählt ist.

**44.** Zusammensetzung nach Anspruch 42 oder 43, **dadurch gekennzeichnet, dass** das ergänzende Monomer ausgewählt ist aus:

a) hydrophilen Monomeren, wie:

· Monomeren mit einer oder mehreren ethylenisch ungesättigten Bindungen, die mindestens eine Carbonsäurefunktion oder Sulfonsäurefunktion enthalten, wie beispielsweise: Acrylsäure, Methacrylsäure, Crotonsäure Maleinsäureanhydrid, Itaconsäure, Fumarsäure, Maleinsäure, Acrylamidopropansulfonsäure,

Vinylbenzoesäure, Vinylphosphorsäure, und deren Salzen,
· Monomeren mit einer oder mehreren ethylenisch ungesättigten Bindungen, die mindestens eine tertiäre Aminofunktion enthalten, wie beispielsweise 2-Vinylpyridin, 4-Vinylpyridin, Dimethylaminoethylmethacrylat, Diethylaminoethylmethacrylat und Dimethylaminopropylmethacrylamid, und deren Salzen,
· Methacrylaten der Formel $CH_2=C(CH_3)-COOR_6$,
worin $R_6$ eine lineare oder verzweigte Alkylgruppe mit 1 bis 4 Kohlenstoffatomen bedeutet, wie Methyl, Ethyl, Propyl oder Isobutyl, wobei die Alkylgruppe mit einem oder mehreren Substituenten substituiert ist, die unter den Hydroxygruppen (z.B. 2-Hydroxypropylmethacrylat und 2-Hydroxyethylmethacrylat) und Halogenatomen (Cl, Br, I, F), wie Trifluorethylmethacrylat, ausgewählt sind,
· Methacrylaten der Formel $CH_2=C(CH_3)-COOR_9$,
worin $R_9$ eine lineare oder verzweigte Alkylgruppe mit 6 bis 12 Kohlenstoffatomen bedeutet, in die optional ein oder mehrere Heteroatome, die unter O, N und S ausgewählt sind, eingebaut ist (sind), wobei die Alkylgruppe mit einem oder mehreren Substituenten substituiert ist, die unter den Hydroxygruppen und Halogenatomen (Cl, Br, I, F) ausgewählt sind,
· Acrylaten der Formel $CH_2=CHCOOR_{10}$,
worin $R_{10}$ eine lineare oder verzweigte Alkylgruppe mit 1 bis 12 Kohlenstoffatomen bedeutet, die mit einem oder mehreren Substituenten substituiert ist, die unter den Hydroxygruppen und den Halogenatomen (Cl, Br, I und F) ausgewählt sind, wie 2-Hydroxypropylacrylat und 2-Hydroxyethylacrylat, oder $R_{10}$ eine Gruppe Alkyl($C_{1-12}$)-O-POE (Polyoxyethylen) bedeutet, wobei die Oxyethyleneinheit 5 bis 30 Mal wiederholt wird, beispielsweise Methoxy-POE, oder $R_{10}$ eine Polyoxyethylengruppe bedeutet, die 5 bis 30 Ethylenoxideinheiten aufweist, und

b) ethylenisch ungesättigten Monomeren, die ein oder mehrere Siliciumatome enthalten, wie Methacryloxypropyltrimethoxysilan und Methacryloxypropyltris(trimethylsiloxy)silan,
und deren Gemischen.

**45.** Zusammensetzung nach einem der Ansprüche 42 oder 43, **dadurch gekennzeichnet, dass** die erste und die zweite Sequenz jede zumindest ein zusätzliches Monomer enthält, das unter Acrylsäure, (Meth)acrylsäure und Trifluorethylmethacrylat und deren Gemischen ausgewählt ist.

**46.** Zusammensetzung nach Anspruch 42 oder 43, **dadurch gekennzeichnet, dass** die erste und die zweite Sequenz jede zumindest ein zusätzliches Monomer enthält, das unter (Meth)acrylsäureestern ausgewählt ist, und optional mindestens ein ergänzendes Monomer, wie (Meth)acrylsäure, und deren Gemische.

**47.** Zusammensetzung nach einem der Ansprüche 42 oder 43, **dadurch gekennzeichnet, dass** die erste und die zweite Sequenz jede vollständig von mindestens einem Monomer abgeleitet ist, das unter (Meth)acrylsäureestern ausgewählt ist, und optional mindestens einem ergänzenden Monomer, wie (Meth)acrylsäure, und deren Gemischen.

**48.** Zusammensetzung nach einem der Ansprüche 42 bis 47, **dadurch gekennzeichnet, dass** das oder die zusätzliche(n) Monomer(e) 1 bis 30 Gew.-% des Gesamtgewichts der ersten und/oder zweiten Sequenz ausmachen.

**49.** Zusammensetzung nach einem der vorhergehenden Ansprüche, **dadurch gekennzeichnet, dass** die Differenz der Glasübergangstemperaturen (Tg) der ersten und der zweiten Sequenz größer als 10 °C, besser größer als 20 °C, vorzugsweise größer als 30 °C und besonders bevorzugt größer als 40 °C ist.

**50.** Zusammensetzung nach einem der vorhergehenden Ansprüche, **dadurch gekennzeichnet, dass** das Sequenzpolymer einen Polydispersitätsindex im Bereich von 2,8 bis 6 aufweist.

**51.** Zusammensetzung nach einem der vorhergehenden Ansprüche, **dadurch gekennzeichnet, dass** das Sequenzpolymer eine gewichtsmittlere Molmasse (Mw) von kleiner oder gleich 300 000 aufweist.

**52.** Zusammensetzung nach dem vorhergehenden Anspruch, **dadurch gekennzeichnet, dass** die gewichtsmittlere Molmasse (Mw) im Bereich von 35 000 bis 200 000 und noch besser im Bereich von 45 000 bis 150 000 liegt.

**53.** Zusammensetzung nach dem vorhergehenden Anspruch, **dadurch gekennzeichnet, dass** die zahlenmittlere Molmasse (Mn) kleiner oder gleich 70 000 ist.

**54.** Zusammensetzung nach einem der Ansprüche 51 bis 53, wobei die zahlenmittlere Molmasse (Mn) im Bereich von 10 000 bis 60 000 und noch besser im Bereich von 12 000 bis 50 000 liegt.

**55.** Zusammensetzung nach einem der vorhergehenden Ansprüche, **dadurch gekennzeichnet, dass** der bei 20° gemessene mittlere Glanz der Zusammensetzung größer oder gleich 30, besser größer oder gleich 35, noch besser größer oder gleich 40, noch besser größer oder gleich 45, noch besser größer oder gleich 50 von 100, noch besser größer oder gleich 55, noch besser größer oder gleich 60, noch besser größer oder gleich 65, noch besser größer oder gleich 70 und noch besser größer oder gleich 75 von 100 ist.

**56.** Zusammensetzung nach einem der vorhergehenden Ansprüche, **dadurch gekennzeichnet, dass** der bei 60° gemessene mittlere Glanz der Zusammensetzung nach dem Verteilen auf einem Träger größer oder gleich 50, noch besser größer oder gleich 60, noch besser größer oder gleich 65, noch besser größer oder gleich 70, noch besser größer oder gleich 75, noch besser größer oder gleich 80, noch besser größer oder gleich 85 und noch besser größer oder gleich 90 von 100 ist.

**57.** Zusammensetzung nach einem der vorhergehenden Ansprüche, **dadurch gekennzeichnet, dass** der bei 20° gemessene mittlere Glanz der Zusammensetzung größer oder gleich 35, vorzugsweise 40, 45 oder 50 auf 100 ist und/oder der bei 60° gemessene mittlere Glanz der Zusammensetzung auf 100 größer oder gleich 65, 70 oder 75 ist.

**58.** Zusammensetzung nach einem der vorhergehenden Ansprüche, **dadurch gekennzeichnet, dass** der bei 20° gemessene Glanz der Zusammensetzung größer oder gleich 60, vorzugsweise 65, 70 oder 75 auf 100 ist und/oder der bei 60° gemessene Glanz der Zusammensetzung auf 100 80, 85 oder 90 ist.

**59.** Zusammensetzung nach einem der vorhergehenden Ansprüche, **dadurch gekennzeichnet, dass** sie 0,1 bis 60 Gew.-% wirksame Substanz des Polymers, vorzugsweise 5 bis 50 Gew.-% und noch bevorzugter 10 bis 40 Gew.-% enthält.

**60.** Kosmetische Zusammensetzung nach einem der vorhergehenden Ansprüche, **dadurch gekennzeichnet, dass** sie ferner ein oder mehrere Farbmittel enthält, die unter den wasserlöslichen Farbstoffen und den pulverförmigen Farbmitteln, wie Pigmenten, Perlglanzstoffen und Pailletten ausgewählt ist.

**61.** Kosmetische Zusammensetzung nach einem der vorhergehenden Ansprüche, **dadurch gekennzeichnet, dass** sie als Suspension, Dispersion, Lösung, Gel, Emulsion und insbesondere Öl-in-Wasser-Emulsion (O/W) oder Wasser-in-Öl-Emulsion (W/O) oder multiple Emulsion (W/O/W oder Polyol/O/W oder O/W/O), als Creme, Schaum, Vesikeldispersion insbesondere von ionischen oder nichtionischen Lipiden, zweiphasige oder mehrphasige Lotion, Paste, insbesondere weiche Paste oder wasserfreie Paste, vorliegt.

**62.** Kosmetische Zusammensetzung nach einem der vorhergehenden Ansprüche, **dadurch gekennzeichnet, dass** sie in wasserfreier Form vorliegt.

**63.** Kosmetische Zusammensetzung nach einem der vorhergehenden Ansprüche, **dadurch gekennzeichnet, dass** es sich um eine Zusammensetzung zum Schminken oder zur Pflege von Keratinsubstanzen handelt.

**64.** Zusammensetzung nach einem der vorhergehenden Ansprüche, **dadurch gekennzeichnet, dass** es sich um ein Produkt zum Schminken der Lippen handelt.

**65.** Zusammensetzung nach einem der vorhergehenden Ansprüche, **dadurch gekennzeichnet, dass** es sich um ein Produkt zum Schminken der Augen handelt.

**66.** Zusammensetzung nach einem der vorhergehenden Ansprüche, **dadurch gekennzeichnet, dass** es sich um ein Produkt zum Schminken der Nägel handelt.

**67.** Kosmetische Einheit umfassend:

a) einen Behälter, der mindestens eine Abteilung abgrenzt, wobei der Behälter mit einer Verschlusseinrichtung verschlossen ist, und
b) eine Zusammensetzung, die sich im Inneren der Abteilung befindet, wobei die Zusammensetzung einer Zusammensetzung nach einem der vorhergehenden Ansprüche entspricht.

**68.** Kosmetische Einheit nach dem vorhergehenden Anspruch, **dadurch gekennzeichnet, dass** der Behälter zumindest zum Teil aus mindestens einem thermoplastischen Material gebildet ist.

**69.** Kosmetische Einheit nach Anspruch 67, **dadurch gekennzeichnet, dass** der Behälter zumindest zum Teil aus mindestens einem nichtthermoplastischen Material und insbesondere aus Glas oder Metall gebildet ist.

**70.** Einheit nach einem der Ansprüche 67 bis 69, **dadurch gekennzeichnet, dass** in der geschlossenen Stellung des Behälters die Verschlusseinrichtung mit dem Behälter verschraubt ist.

**71.** Einheit nach einem der Ansprüche 67 bis 69, **dadurch gekennzeichnet, dass** in der geschlossenen Stellung des Behälters die Verschlusseinrichtung an den Behälter auf eine andere Weise als durch Verschrauben gekoppelt ist, insbesondere durch Einrasten, Kleben oder Schweißen.

**72.** Einheit nach einem der Ansprüche 67 bis 71, **dadurch gekennzeichnet, dass** die Zusammensetzung im Inneren der Abteilung in etwa unter Atmosphärendruck steht.

**73.** Einheit nach einem der Ansprüche 67 bis 71, **dadurch gekennzeichnet, dass** die Zusammensetzung im Inneren der Abteilung unter Druck steht.

**74.** Kosmetisches Verfahren zum Schminken oder für die Pflege von Keratinsubstanzen, das umfasst, eine kosmetische Zusammensetzung nach einem der Ansprüche 1 bis 66 auf die Keratinsubstanzen aufzutragen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6153206 A
- FR 2809306 A
- WO 03046032 A
- EP 0955039 A
- US 4887622 A
- FR 2796529
- FR 2722380
- US 5492426 A
- FR 2761959
- WO 0103538 A
- WO 03018423 A
- FR 2791042
- FR 2792618


### Littérature non-brevet citée dans la description


- Polymer Handbook. John Wiley, 1989